(19) Europäisches Patentamt
European Patent Office
Office européen des brevets

(11) EP 2 286 817 A2

(12) EUROPEAN PATENT APPLICATION

(43) Date of publication:
**23.02.2011 Bulletin 2011/08**

(21) Application number: **10075488.6**

(22) Date of filing: **13.01.2004**

(51) Int Cl.:
***A61K 31/519*** (2006.01) ***A61K 31/505*** (2006.01)
***A61K 31/55*** (2006.01) ***A61K 31/551*** (2006.01)
***A61K 45/06*** (2006.01) ***A61P 1/00*** (2006.01)

(84) Designated Contracting States:
**AT BE BG CH CY CZ DE DK EE ES FI FR GB GR HU IE IT LI LU MC NL PT RO SE SI SK TR**

(30) Priority: **13.01.2003 US 440077 P**
**04.08.2003 US 492480 P**

(62) Document number(s) of the earlier application(s) in accordance with Art. 76 EPC:
**04701811.4 / 1 558 081**

(71) Applicant: **Edusa Pharmaceuticals, Inc**
**Princeton NJ 08540 (US)**

(72) Inventor: **Landau, Steven B**
**Massachusetts 02481 (US)**

(74) Representative: **Snodin, Michael D.**
**Potter Clarkson LLP**
**Park View House**
**58 The Ropewalk**
**GB-Nottingham NG1 5DD (GB)**

Remarks:
This application was filed on 22-09-2010 as a divisional application to the application mentioned under INID code 62.

# (54) Method of treating functional bowel disorders

(57) The invention relates to a method of treating functional bowel disorders in a subject in need of treatment. The method comprises administering to a subject in need of treatment a therapeutically effective amount of a compound that has 5-$HT_3$ receptor antagonist activity and NorAdrenaline Reuptake Inhibitor (NARI) activity. The invention further relates to a method of treating a functional bowel disorder in a subject in need thereof, comprising coadministering to said subject a first amount of a 5-$HT_3$ antagonist and a second amount of a NARI, wherein the first and second amounts together comprise a therapeutically effective amount or are each present in a therapeutically effective amount. In addition, the method of the invention comprises administering a NARI alone. The functional bowel disorders which can be treated according to the method of the invention include IBS, functional abdominal bloating, functional constipation and functional diarrhea.

EP 2 286 817 A2

## Description

RELATED APPLICATIONS

This application claims the benefit of U.S. Provisional Application No. 60/492,480 filed on August 4, 2003 and U.S. Provisional Application No. 60/440,077 filed on January 13, 2003. The entire teachings of the above applications are incorporated herein by reference.

BACKGROUND OF THE INVENTION

Functional Bowel Disorders (FBDs) are functional gastrointestinal disorders having symptoms attributable to the mid or lower gastrointestinal tract. FBDs can include, Irritable Bowel Syndrome (IBS), functional abdominal bloating, functional constipation and functional diarrhea (see, for example, Thompson et al., Gut, 45 (Suppl. II):II43-II47 (1999)). Of these disorders, IBS alone accounts for up to about 3.5 million physician visits per year, and is the most common diagnosis made by gastroenterologists, accounting for about 25% of all patients (Camilleri and Choi, Aliment. Pharm. Ther., 11:3-15 (1997)). Overall, it is estimated that IBS affects up to 20% of the adult population worldwide with only 10-50% of those afflicted with IBS actually seeking medical attention. Women apparently are more often affected than men. In addition, psychological factors, for example, emotional stress or overt psychological disease, modulate and exacerbate the physiological mechanisms that operate in IBS.

Due to a lack of readily identifiable structural or biochemical abnormalities in IBS, the medical community has developed a consensus definition and criteria, known as the Rome II Criteria, to aid in diagnosis of IBS. Therefore, diagnosis of IBS is one of exclusion and is based on the observed symptoms in any given case. The Rome II criteria for IBS, include at least 12 weeks in the preceding 12 months, which need not be consecutive, of abdominal pain or discomfort that has two of three features:

(1) Relieved with defecation; and/or
(2) Onset associated with a change in the frequency of stools; and/or
(3) Onset associated with a change in form (appearance) of stool.

Other symptoms, such as abnormal stool frequency, abnormal stool form, abnormal stool passage, passage of mucus, and/or bloating or feeling of abdominal distension, cumulatively support the diagnosis of IBS.

Further, subjects with IBS exhibit visceral hypersensitivity, the presence of which behavioral studies have shown is the most consistent abnormality in IBS. For example, patients and controls were evaluated for their pain thresholds in response to progressive distension of the sigmoid colon induced by a balloon. At the same volume of distension, the patients reported higher pain scores compared to controls. This finding has been reproduced in many studies and with the introduction of the barostat, a computerized distension device, the distension procedures have been standardized. Two concepts of visceral hypersensitivity, hyperalgesia and allodynia, have been introduced. More specifically, hyperalgesia refers to the situation in which normal visceral sensations are experienced at lower intraluminal volumes. While for a finding of allodynia, pain or discomfort is experienced at volumes usually producing normal internal sensations (see, for example, Mayer E.A. and Gebhart, G.F., Basic and Clinical Aspects of Chronic Abdominal Pain, Vol 9, 1st ed. Amsterdam: Elsevier, 1993:3-28).

As such, IBS is a functional bowel disorder in which abdominal pain or discomfort is associated with defecation or a change in bowel habit. Therefore, IBS has elements of an intestinal motility disorder, a visceral sensation disorder, and a central nervous disorder. While the symptoms of IBS have a physiological basis, no physiological mechanism unique to IBS has been identified. In some cases, the same mechanisms that cause occasional abdominal discomfort in healthy individuals operate to produce the symptoms of IBS. The symptoms of IBS are therefore a product of quantitative differences in the motor reactivity of the intestinal tract, and increased sensitivity to stimuli or spontaneous contractions.

Conventional treatments for IBS are based on the severity and the nature of the symptoms being experienced by the patient and whether any psychological factors are involved. Treatment of IBS may include one or more of the following: lifestyle changes, pharmacological treatment and psychological treatment. However, there is no general treatment which is applicable to all cases of IBS.

In certain cases, the exclusion of foods which aggravate IBS symptoms is recommended. However, this type of treatment is only effective when the underlying or contributing cause of IBS is related to diet. Psychological treatment can be used in the treatment of IBS. Again, however, this treatment does not provide a universal cure for the symptoms of IBS since not all cases of IBS are due to psychological factors.

Pharmacologically active agents are often used to treat IBS. Anti-diarrheals, such as loperamide, diphenoxylate, and codeine phosphate, for diarrhea-predominant IBS; and antispasmodic agents, such as anticholinergics and smooth muscle relaxants, such as cimetropium bromide, pinaverium bromide, octilium bromide, trimebutine, and mebeverine,

for diarrhea-predominant IBS and abdominal pain. Again, while the antichloinergics and smooth muscle relaxants provide some pain relief, their effects on other symptoms associated with IBS is unclear.

Tricyclic antidepressants, such as amitriptyline, imipramine, and doxepin, are frequently used to treat IBS. The tricyclics have been selected for use in IBS based on the anticholinergic and analgesic properties which they possess, which are independent of their psychotropic effects. It has been reported that the anticholinergic and analgesic effects of the tricyclic antidepressants on the gastrointestinal tract occur within 24 to 48 hours and that the tricyclic antidepressants can benefit patients with pain predominant IBS and increased bowel frequency (see, Clouse, R.R., Dig. Dis. Sci., 39: 2352-2363 (1994)).

However, the undesirable side effects associated with the use of tricyclic antidepressants to treat IBS are a significant drawback for this therapy. For example, the anticholinergic properties of the tricyclic antidepressants can cause dry mouth, constipation, blurred vision, urinary retention, weight gain, hypertension and cardiac side effects, such as palpitations and arrhythmia.

Further, many patients are reluctant to undergo treatment for IBS with a drug typically administered for the treatment of depression. That is, although the tricyclic antidepressants are prescribed for use in IBS because of their anticholinergic and analgesic properties, this distinction is not appreciated by the general population (e.g., those outside the medical community) and the stigma attached with use of tricyclic antidepressants continues.

Furthermore, the newer antidepressants, in particular the selective serotonin reuptake inhibitors, such as fluoxetine, sertraline, and paroxetine, have not been shown to be more effective than the tricyclic antidepressants, although some evidence suggests these compounds may have fewer side effects.

Central Nervous System (CNS) treatments have received attention as potential IBS therapies because of the relationship between the CNS and the neural networks within the walls of the gut, the latter of which form the Enteric Nervous System (see, e.g., Wood et al., Gut, 45 (Suppl II):II6-II16 (1999)). The use of 5-$HT_3$ receptor antagonists has been proposed as a possible treatment for IBS. The 5-$HT_3$ receptors are ligand-gated ion channels that elicit the depolarizing actions of serotonin (5-hydroxytryptamine, 5-HT), which facilitate neurotransmitter release. In the gastrointestinal tract, 5-$HT_3$ receptors are located on postsynaptic enteric neurons and on afferent sensory fibers. 5-$HT_3$ receptors are also found in dorsal root ganglion neurons conveying sensory information from the distal gastrointestinal tract to the spinal cord. Antagonism of these receptors has been found to reduce visceral pain, retard colonic transit and enhance small intestinal absorption.

In fact, clinical pharmacology studies have shown that 5-$HT_3$ receptor antagonists slow whole gut transit time in healthy volunteers, enhance colonic compliance and reduce perception of volume based distension in patients with IBS and retard transit through the colon in patients with symptoms of diarrhea. However, constipation and sequelae which have resulted in colonic surgery, as well as acute ischemic colitis have been significant adverse events with the use of the 5-$HT_3$ receptor antagonist alosetron for the treatment of IBS. For example, complications of this type, some fatal, resulted in the temporary withdrawal from the US market of the 5-$HT_3$ receptor antagonist, alosetron, for the treatment of IBS.

In view of the above, there is a need for improved treatment of functional bowel disorders, particularly for the treatment of IBS.

## SUMMARY OF THE INVENTION

The invention relates to a method of treating a functional bowel disorder in a subject in need of treatment. The method comprises administering to a subject in need of treatment a therapeutically effective amount of a compound that has 5-$HT_3$ receptor antagonist activity and NorAdrenaline Reuptake Inhibitor (NARI) activity. The functional bowel disorder can be selected from IBS, functional abdominal bloating, functional constipation and functional diarrhea.

In a particular embodiment, the compounds having 5-$HT_3$ receptor antagonist activity and NARI activity are thieno[2,3-d]pyrimidine derivatives such as those described in U.S. Patent No. 4,695,568, the entire content of which is incorporated herein by reference.

In a specific embodiment, the compounds having 5-$HT_3$ receptor antagonist activity and NARI activity are represented by structural Formula I:

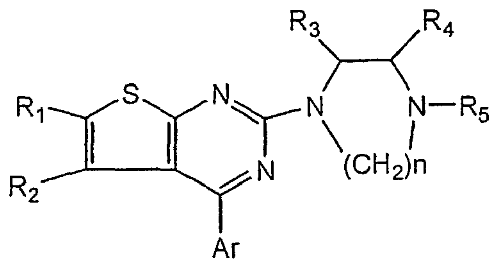

I

wherein, $R_1$ and $R_2$ independently represent hydrogen, halogen or a $C_1$-$C_6$ alkyl group; or $R_1$ and $R_2$ together with the carbon atoms to which they are attached form a cycloalkylene group having 5 to 6 carbon atoms;
$R_3$ and $R_4$ independently represent hydrogen or a $C_1$-$C_6$ alkyl group;
$R_5$ is hydrogen, $C_1$-$C_6$ alkyl,

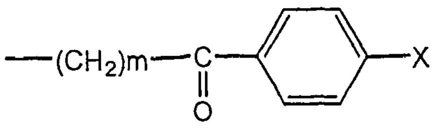

,

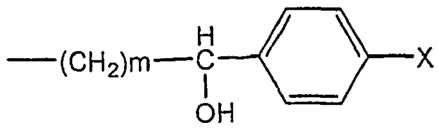

or -C(O)-NH-$R_6$ ,

wherein m is an integer from about 1 to about 3, X is halogen and $R_6$ is a $C_1$-$C_6$ alkyl group;

Ar is a substituted or unsubstituted phenyl, 2-thienyl or 3-thienyl group; and n is 2 or 3; or a pharmaceutically acceptable salt thereof.

In a specific embodiment, the compound having 5-$HT_3$ receptor antagonist activity and NARI activity is represented by the formula:

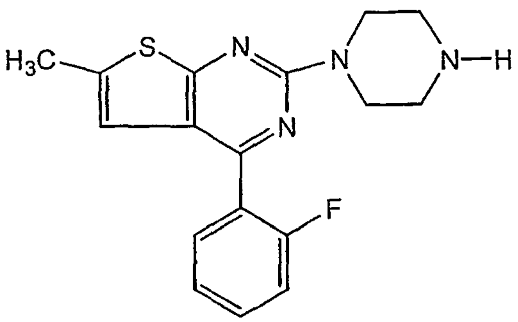

II

or a pharmaceutically acceptable salt thereof. This compound is commonly referred to as MCI-225 or DDP-225. The chemical name of the structure set forth in the formula is: 4-(2-fluorophenyl)-6-methyl-2-(1-piperazinyl)thieno[2,3-d] pyrimidine.

In a certain embodiment, the functional bowel disorder is IBS. In a particular embodiment, the IBS is diarrhea predominant IBS. In another embodiment, the IBS is alternating constipation/diarrhea IBS. In a further embodiment, the IBS is nonconstipated IBS.

The invention further relates to a method of treating a functional bowel disorder in a subject in need thereof, comprising coadministering to said subject a therapeutically effective amount of a 5-$HT_3$ receptor antagonist and a therapeutically effective amount of a NARI. The functional bowel disorder can be selected from IBS, functional abdominal bloating, functional constipation and functional diarrhea.

The invention further relates to a method of treating a functional bowel disorder in a subject in need thereof, comprising coadministering to said subject a first amount of a 5-$HT_3$ receptor antagonist and a second amount of a

NARI, wherein the first and second amounts together comprise a therapeutically effective amount. The functional bowel disorder can be selected from IBS, functional abdominal bloating, functional constipation and functional diarrhea.

In a specific embodiment, the coadministration methods can be used to treat IBS. In a particular embodiment, the IBS is diarrhea predominant IBS. In another embodiment, the IBS is alternating constipation/diarrhea IBS. In a further embodiment, the IBS is nonconstipated IBS.

In addition, the invention relates to a method of treating functional bowel disorder in a subject in need thereof comprising administering a therapeutically effective amount of a NARI. In this embodiment, the NARI is characterized by the substantial absence of anticholinergic effects. The functional bowel disorder can be selected from IBS, functional abdominal bloating, functional constipation and functional diarrhea.

In a specific embodiment, the administration of the NARI can be used to treat IBS. In a particular embodiment, the IBS is diarrhea predominant IBS. In another embodiment, the IBS is alternating constipation/diarrhea IBS. In a further embodiment, the IBS is nonconstipated IBS.

The invention further relates to pharmaceutical compositions useful for the treatment of a functional bowel disorder. The pharmaceutical composition comprises a first amount of a 5-$HT_3$ receptor antagonist compound and a second amount of a NARI compound. The pharmaceutical compositions of the present invention can optionally contain a pharmaceutically acceptable carrier. The 5-$HT_3$ receptor antagonist and the NARI can each be present in the pharmaceutical composition in a therapeutically effective amount. In another aspect, said first and second amounts can together comprise a therapeutically effective amount.

The pharmaceutical composition can be used to treat a functional bowel disorder, such as a functional bowel disorder selected from the group consisting of IBS, functional abdominal bloating, functional constipation and functional diarrhea. In a certain embodiment, the functional bowel disorder is IBS. In a particular embodiment, the IBS is diarrhea predominant IBS. In another embodiment, the IBS is alternating constipation/diarrhea IBS. In a further embodiment, the IBS is nonconstipated IBS.

The invention further relates to use of a compound that has 5-$HT_3$ receptor antagonist activity and NARI activity for the manufacture of a medicament for treating a functional bowel disorder. In addition, the invention also relates to the use of a pharmaceutical composition comprising a first amount of a 5-$HT_3$ receptor antagonist compound and a second amount of a NARI compound for the manufacture of a medicament for the treatment of a functional bowel disorder. The pharmaceutical composition used for the manufacture of a medicament for treating a functional bowel disorder can optionally contain a pharmaceutically acceptable carrier. The 5-$HT_3$ receptor antagonist and the NARI can each be present in the pharmaceutical composition in a therapeutically effective amount or said first and second amounts can together comprise a therapeutically effective amount. Further, the invention relates to the use of a NARI for the manufacture of a medicament for treating a functional bowel disorder.

The foregoing and other objects, features and advantages of the invention will be apparent from the following more particular description of preferred embodiments of the invention.

BRIEF DESCRIPTION OF THE DRAWINGS

FIG. 1 is a graph of visceromotor response (number of abdominal muscle contractions) recorded during 10 min periods of colorectal distension versus distension pressure for untreated (no vehicle or drug administered) sensitized and non-sensitized male rats and for sensitized male rats treated with MCI-225.

FIG. 2 is a graph of visceromotor response (number of abdominal muscle contractions) recorded during 10 min periods of colorectal distension versus distension pressure for untreated (no vehicle or drug administered) sensitized and non-sensitized male rats and for sensitized male rats treated with vehicle alone.

FIG. 3 is a graph of visceromotor response (number of abdominal muscle contractions) recorded during 10 min periods of colorectal distension versus distension pressure for sensitized male rats treated with 1 mg/kg, 5 mg/kg or 10 mg/kg of ondansetron i.p. or vehicle alone.

FIG. 4 is a graph of visceromotor response (number of abdominal muscle contractions) recorded during 10 min periods of colorectal distension versus distension pressure for sensitized male rats treated with 3 mg/kg, 10 mg/kg or 30 mg/kg of nisoxetine i.p. or vehicle alone.

FIG. 5 is a graph of visceromotor response (number of abdominal muscle contractions) recorded during 10 min periods of colorectal distension versus distension pressure for sensitized male rats treated with 3 mg/kg, 10 mg/kg or 30 mg/kg of MCI-225 i.p. or vehicle alone.

FIG. 6 is a bar graph of fecal pellet output per hour for male rats in the indicated control groups of the Water Avoidance Stress (WAS) Model.

FIG. 7 is a bar graph of fecal pellet output per hour for male rats subjected to the WAS Model and treated with 3 mg/kg, 10 mg/kg or 30 mg/kg MCI-225 or vehicle alone.

FIG. 8 is a bar graph of fecal pellet output per hour for male rats subjected to the WAS Model and treated with 3 mg/kg, 10 mg/kg or 30 mg/kg nisoxetine or vehicle alone.

FIG. 9 is a bar graph of fecal pellet output per hour for male rats subjected to the WAS Model and treated with 1 mg/kg, 5 mg/kg or 10 mg/kg of ondansetron or vehicle alone.

FIG. 10 is a bar graph of fecal pellet output per hour for male rats subjected to the WAS Model and treated with a combination of ondansetron (10 mg/kg) and nisoxetine (30 mg/kg).

FIG. 11 is a bar graph of Percent Small Intestinal Transit for male rats in the indicated control groups (untreated; vehicle (propylene glycol)) of the Small Intestinal Transit (SIT) Rodent Model.

FIG. 12 is a bar graph of Percent Small Intestinal Transit for male rats subjected to the SIT Rodent Model and treated with 3 mg/kg, 10 mg/kg or 30 mg/kg MCI-225 i.p. or vehicle alone.

FIG. 13 is a bar graph of Percent Small Intestinal Transit for male rats subjected to the SIT Rodent Model and treated with 3 mg/kg, 10 mg/kg or 30 mg/kg nisoxetine i.p. or vehicle alone.

FIG. 14 is a bar graph of Percent Small Intestinal Transit for male rats subjected to the SIT Rodent Model and treated with 1 mg/kg, 5 mg/kg or 10 mg/kg ondansetron i.p. or vehicle alone.

FIG. 15 is a bar graph of Percent Small Intestinal Transit for male rats subjected to the SIT Rodent Model and treated with a combination of nisoxetine (10 mg/kg) and ondansetron (5 mg/kg) or vehicle alone.

## DETAILED DESCRIPTION OF THE INVENTION

The invention relates to methods of treating a functional bowel disorder in a subject in need of treatment. In particular, the invention relates to method of treating IBS in a subject in need of treatment.

### MONOAMINE NEUROTRANSMITTERS:

Monoamine neurotransmitters such as noradrenaline (also referred to as norepinephrine), serotonin (5-hydroxytryptamine, 5-HT) and dopamine are known and disturbances in these neurotransmitters have been indicated in many types of disorders, such as depression. These neurotransmitters travel from the terminal of a neuron across a small gap referred to as the synaptic cleft and bind to receptor molecules on the surface of a second neuron. This binding elicits intracellular changes that initiate or activate a response or change in the postsynaptic neuron. Inactivation occurs primarily by transport of the neurotransmitter back into the presynaptic neuron, which is referred to as reuptake. These neurons can be found both in the Central Nervous System (CNS) and in the Enteric Nervous System (ENS).

### NORADRENALIN AND NORADRENALINE REUPTAKE INHIBITORS:

As used herein, the term NorAdrenaline Reuptake Inhibitor (NARI) refers to an agent (e.g., a molecule, a compound) which can inhibit noradrenaline transporter function. For example, a NARI can inhibit binding of a ligand of a noradrenaline transporter to said transporter and/or inhibit transport (e.g., uptake or reuptake of noradrenaline). As such, inhibition of the noradrenaline transport function in a subject, can result in an increase in the concentration of physiologically active noradrenaline. It is understood that NorAdrenergic Reuptake Inhibitor and NorEpinephrine Reuptake Inhibitor (NERI) are synonymous with NorAdrenaline Reuptake Inhibitor (NARI).

As used herein, noradrenaline transporter refers to naturally occurring noradrenaline transporters (e.g., mammalian noradrenaline transporters (e.g., human *(Homo sapiens)* noradrenaline transporters, murine (e.g., rat, mouse) noradrenaline transporters)) and to proteins having an amino acid sequence which is the same as that of a corresponding naturally occurring noradrenaline transporter (e.g., recombinant proteins). The term includes naturally occurring variants, such as polymorphic or allelic variants and splice variants.

In certain embodiments, the NARI can inhibit the binding of a ligand (e.g., a natural ligand such as noradrenaline, or other ligand such as nisoxetine) to a noradrenaline transporter. In other embodiments, the NARI can bind to a noradrenaline transporter. For example, in a preferred embodiment, the NARI can bind to a noradrenaline transporter, thereby inhibiting binding of a ligand to said transporter and inhibiting transport of said ligand. In another preferred embodiment, the NARI can bind to a noradrenaline transporter, and thereby inhibit transport.

The NARI activity of a compound can be determined employing suitable assays. More specifically, to determine the inhibition constant (Ki) for noradrenaline reuptake, an assay which monitors inhibition of noradrenaline (NA) uptake can be used. For example, radiolabelled noradrenaline, such as [$^3$H]NA and the test compound of interest can be incubated under conditions suitable for uptake with brain tissue or a suitable fraction thereof, for example, a synaptosomal fraction from rat brain tissue (harvested and isolated in accordance with generally accepted techniques), and the amount of uptake of [$^3$H]NA in the tissue or fraction can be determined (e.g., by liquid scintillation spectrometry). $IC_{50}$ values can be calculated by nonlinear regression analysis. The inhibition constants, Ki values, can then be calculated from the $IC_{50}$ values using the Cheng-Prusoff equation:

$$K_i = \frac{IC_{50}}{1 + ([L]/K_d)}$$

wherein [L] = the concentration of free radioligand used in the assay and $K_d$ = the equilibrium dissociation constant of the radioligand. To determine the non-specific uptake, incubations can be performed by following the same assay, but in the absence of test compound at 4°C (i.e., under conditions not suitable for uptake).

In a preferred embodiment, NARI activity is determined using the radioligand uptake assay described above, according to the procedure detailed in Eguchi et al., Arzneim.-Forschung/Drug Res., 47(12): 1337-47 (1997).

Specifically, rats are decapitated and the cortical, hypothalamic, hippocampal and striatal tissues are rapidly dissected. The tissues are homogenized (Potter homogenizer with Teflon pestle) in 10 volumes of ice cold 0.32 mol/L sucrose. The $P_2$ fraction is obtained by centrifugation at 1000 x g for 10 minutes and 11500 x g for 20 minutes and suspended in Krebs-Ringer phosphate buffer, pH 7.4 (124 mmol/L NaCl, 5 mmol/L KCl, 20 mmol/L $Na_2HPO_4$, 1.2 mmol/L $KH_2PO_4$, 1.3 mmol/L $MgSO_4$, 0.75 mmol/L $CaCl_2$, 10 mmol/L glucose). The [$^3$H]NA uptake assays are performed on the cortical and hypothalamic synaptosomes.

The assay tubes contain radiolabled noradrenaline, [$^3$H]NA, in a volume of 0.2 mL, compounds at 5 or more concentrations in a volume of 0.1 mL, and the oxygenated buffer described above in a volume of 0.5 mL. After 5 minutes preincubation at 37°C, uptake is initiated by the addition of the synaptosomal fraction in volume of 0.2 mL. The final concentration of [$^3$H]NA in the incubation mixtures is 0.25 μmol/L. The reaction is stopped after 5 minutes by filtration through Whatman GF/B glass fiber filter under a vacuum with a cell harvester. The filter is rinsed three times with 4 mL of saline and placed in a scintillation vial containing 10 mL of Atomlight (Du Pont/NEN Research Products). Radioactivity is measured by liquid scintillation spectrometry. For determination of non-specific uptake, incubations are performed at 4 °C without the addition of test compounds. $IC_{50}$ values are calculated by nonlinear regression analysis. Inhibitor constants, Ki values, are calculated from the $IC_5$. values using the Cheng-Prusoff equation.

NARI compounds suitable for use in the invention have a Ki value for NARI activity of about 500 nmol/L or less, such as about 250 nmol/L or less, for example, about 100 nmol/L or less. It is preferred that the Ki value for NARI activity be about 100 nmol/L or less. It is understood that the exact value of the Ki for a particular compound can vary depending on the assay conditions employed for determination (e.g., radioligand and tissue source). As such, it is preferred that the NARI activity be assessed essentially according to the radioligand binding assay described in Eguchi et al., Arzneim.-Forschung/Drug Res., 47(12): 1337-47 (1997) and discussed in detail above.

In addition, to possessing sufficient NARI activity, it is preferred that the NARI compounds possess one or more characteristics selected from the group consisting of:

a) the substantial absence of anticholinergic effects;
b) the selective inhibition of noradrenaline reuptake as compared to inhibition of serotonin reuptake; and
c) the selective inhibition of noradrenaline reuptake as compared to inhibition of dopamine reuptake.

Selective inhibition of noradrenaline reuptake as compared to inhibition of serotonin or dopamine reuptake can be determined by comparing the Ki values for the respective reuptake inhibitions. The inhibition constants for serotonin and dopamine reuptake can be determined as described above for nordrenaline, but employing the appropriate radioligand and tissue for the activity being assessed (e.g., [$^3$H] 5-HT for serotonin, using e.g., hypothalamic or cortical tissue and [$^3$H]DA for dopamine (DA), using e.g., striatal tissue).

A preferred method of determining serotonin reuptake inhibition and dopaminergic reuptake inhibition is described in Eguchi et al., Arzneim.-Forschung/Drug Res., 47(12): 1337-47 (1997). Specifically, rats are decapitated and the cortical, hypothalamic, hippocampal and striatal tissues are rapidly dissected. The tissues are homogenized (Potter homogenizer with Teflon pestle) in 10 volumes of ice cold 0.32 mol/L sucrose. The $P_2$ fraction is obtained by centrifugation at 1000 x g for 10 minutes and 11500 x g for 20 minutes and suspended in Krebs-Ringer phosphate buffer, pH 7.4 (124 mmol/L NaCl, 5 mmol/L KCl, 20 mmol/L $Na_2HPO_4$, 1.2 mmol/L $KH_2PO_4$, 1.3 mmol/L $MgSO_4$, 0.75 mmol/L $CaCl_2$, 10 mmol/L glucose). The [$^3$H]5-HT uptake assays are performed on the cortical, hypothalamic and hippocampal synaptosomes, and the [$^3$H]DA uptake assays are performed on striatal synaptosomes.

The assay tubes contain the appropriate radiolabled ligand (i.e., [$^3$H]5-HT or [$^3$H]DA), in a volume of 0.2 mL, compounds at 5 or more concentrations in a volume of 0.1 mL, and the oxygenated buffer described above in a volume of 0.5 mL. After 5 minutes preincubation at 37 °C, uptake is initiated by the addition of the synaptosomal fraction in volume of 0.2 mL. The final concentration of [$^3$H]DA in the striatal incubation mixtures is 0.4 μmol/L. The final concentrations of [$^3$H] 5-HT in the cortical, hypothalamic and hippocampal synaptosome incubation mixtures are 0.02 μmol/L,

0.04 μmol/L and 0.08 μmol/L. The reaction is stopped after 5 minutes ([$^3$H]5-HT) or 3 minutes [$^3$H]DA by filtration through Whatman GFB glass fiber filter under a vacuum with a cell harvester. The filter is rinsed three times with 4 mL of saline and placed in a scintillation vial containing 10 mL of Atomlight (Du Pont/NEN Research Products). Radioactivity is measured by liquid scintillation spectrometry. For determination of non-specific uptake incubations are performed at 4 °C without the addition of test compounds. $IC_{50}$ values are calculated by nonlinear regression analysis. Inhibition constants, Ki values, are calculated from the $IC_{50}$ values using the Cheng-Prusoff equation.

Following determination of the Ki values for inhibition of noradrenaline, serotonin and/or dopamine uptake, the ratio of the activities can be determined. Selective inhibition of noradrenaline reuptake as compared to inhibition of serotonin reuptake and/or dopaminergic reuptake, refers to a compound having a Ki value for inhibition of serotonin (re) uptake and/or dopamine (re)uptake which is about 10 times or more than the Ki for inhibition of noradrenaline (re)uptake. That is, the ratio, Ki inhibition of serotonin (re)uptake / Ki inhibition of noradrenaline (re)uptake, is about 10 or more, such as about 15 or more, about 20 or more, for example, about 30, 40 or 50 or more. Likewise, the ratio, Ki inhibition of dopamine (re)uptake / Ki inhibition noradrenaline (re)uptake, is about 10 or more, such as about 15 or more, about 20 or more, for example, about 30, 40 or 50 or more.

It is preferred that the Ki values for comparison are determined according to the method of Eguchi *et al.,* discussed in detail above. It is most preferred, that the Ki values for NARI activity and inhibition of serotonin reuptake activity, which are compared to determine selective inhibition are determined according to the method of Eguchi *et al.* using a synaptosomal preparation from rat hypothalamic tissue. Further, it is most preferred, that the Ki values for NARI activity and inhibition of dopamine reuptake activity, which are compared to determine selective inhibition are determined according to the method of Eguchi *et al.* using a synaptosomal preparation from rat hypothalamic tissue for inhibition of noradrenaline uptake and from rat striatal tissue for inhibition of dopamine uptake.

In another embodiment, the NARI is characterized by the substantial absence of anticholinergic effects. As used herein, substantial absence of anticholinergic effects, refers to a compound which has an $IC_{50}$ value for binding to muscarinic receptors of about 1 μmol/L or more. The $IC_{50}$ value for binding to muscarinic receptors can be determined using a suitable assay, such as an assay which determines the ability of a compound to inhibit the binding of suitable radioligand to muscarinic receptors. A preferred assay for determination of the $IC_{50}$ value for binding of a compound to muscarinic receptors is described in Eguchi et al., Arzneim.-Forschung/Drug Res., 47(12): 1337-47 (1997).

Specifically, the binding assays for determination of binding to muscarinic receptors can be performed on tissue isolated from the rat cerebral cortex. The buffer is any suitable buffer, for example, 50 mmol/L Tris-HCl, pH=7.4. The preferred radiolabeled ligand is [$^3$H]QNB (3-quinuclidinyl benzilate) which is preset in a final concentration of 0.2 nmol/L. The test compound is added at various concentrations and the resulting mixtures are incubated for 60 minutes at 37 °C. The reaction is terminated by rapid vacuum filtration onto glass fiber filter. Radioactivity trapped on the filter is measured by scintillation spectrometry. Non-specific binding is determined using 100 μmol/L atropine. $IC_{50}$ values can be calculated by nonlinear regression analysis.

In a particular embodiment, the NARI compound can be selected from venlafaxine, duloxetine, buproprion, milnacipran, reboxetine, lefepramine, desipramine, nortriptyline, tomoxetine, maprotiline, oxaprotiline, levoprotiline, viloxazine and atomoxetine.

In a preferred embodiment, the NARI compound can be selected from reboxetine, lefepramine, desipramine, nortriptyline, tomoxetine, maprotiline, oxaprotiline, levoprotiline, viloxazine and atomoxetine.

SETOTONIN AND 5-$HT_3$ RECEPTOR ANTAGONISTS:

The neurotransmitter serotonin was first discovered in 1948 and has subsequently been the subject of substantial scientific research. Serotonin, also referred to as 5-hydroxytryptamine (5-HT), acts both centrally and peripherally on discrete 5-HT receptors. Currently, fourteen subtypes of serotonin receptors are recognized and delineated into seven families, 5-$HT_1$ through 5-$HT_7$. These subtypes share sequence homology and display some similarities in their specificity for particular ligands. A review of the nomenclature and classification of the 5-HT receptors can be found in Neuropharm., 33: 261-273 (1994) and Pharm. Rev., 46:157-203 (1994).

5-$HT_3$ receptors are ligand-gated ion channels that are extensively distributed on enteric neurons in the human gastrointestinal tract, as well as other peripheral and central locations. Activation of these channels and the resulting neuronal depolarization have been found to affect the regulation of visceral pain, colonic transit and gastrointestinal secretions. Antagonism of the 5-$HT_3$ receptors has the potential to influence sensory and motor function in the gut.

As used herein, 5-$HT_3$ receptor refers to naturally occurring 5-$HT_3$ receptors (e.g., mammalian 5-$HT_3$ receptors (e.g., human *(Homo sapiens)* 5-$HT_3$ receptors, murine (e.g., rat, mouse) 5-$HT_3$ receptors)) and to proteins having an amino acid sequence which is the same as that of a corresponding naturally occurring 5-$HT_3$ receptor (e.g., recombinant proteins). The term includes naturally occurring variants, such as polymorphic or allelic variants and splice variants.

As used herein, the term 5-$HT_3$ receptor antagonist refers to an agent (e.g., a molecule, a compound) which can inhibit 5-$HT_3$ receptor function. For example, a 5-$HT_3$ receptor antagonist can inhibit binding of a ligand of a 5-$HT_3$

receptor to said receptor and/or inhibit a 5-$HT_3$ receptor-mediated response (e.g., reduce the ability of 5-$HT_3$ to evoke the von Bezold-Jarisch reflex).

In certain embodiments, the 5-$HT_3$ receptor antagonist can inhibit binding of a ligand (e.g., a natural ligand, such as serotonin (5-$HT_3$), or other ligand such as GR65630) to a 5-$HT_3$ receptor. In certain embodiments, the 5-$HT_3$ receptor antagonist can bind to a 5-$HT_3$ receptor. For example, in a preferred embodiment, the 5-$HT_3$ receptor antagonist can bind to a 5-$HT_3$ receptor, thereby inhibiting the binding of a ligand to said receptor and a 5-$HT_3$ receptor-mediated response to ligand binding. In another preferred embodiment, the 5-$HT_3$ receptor antagonist can bind to a 5-$HT_3$ receptor, and thereby inhibit a 5-$HT_3$ receptor-mediated response.

5-$HT_3$ receptor antagonists can be identified and activity assessed by any suitable method, for example, by a method which assesses the ability of a compound to inhibit radioligand binding to 5-$HT_3$ receptor (see, for example, Eguchi et al., Arzneim.-Forschung/Drug Res., 47(12): 1337-47 (1997) and G. Kilpatrick et al., Nature, 330: 746-748 (1987)) and/or by their effect on the 5-$HT_3$-induced von Bezold-Jarisch (B-J) reflex in the cat or rat (following the general methods described by Butler et al., Br. J. Pharmacol., 94: 397-412 (1988) and Ito et al., J. Pharmacol. Exp. Ther., 280 (1): 67-72 (1997), respectively).

In a preferred embodiment, 5-$HT_3$ receptor antagonist activity of a compound can be determined according to the method described in Eguchi et al., Arzneim.-Forschung/Drug Res., 47(12): 1337-47 (1997). Specifically, the binding assays for determination of binding to the 5-$HT_3$ receptor can be performed on N1E-115 mouse neuroblastoma cells (American Type Culture Collection (ATCC) Accession No. CRL-2263) in 20 mmol/L HEPES buffer (pH=7.4) containing 150 mmol/L NaCl, 0.35 mmol/L of radiolabeled ligand ([$^3$H]GR65630) and the test compound at 6 or more concentrations at 25 °C for 60 minutes. The reaction is terminated by rapid vacuum filtration onto glass fiber filter. Radioactivity trapped on the filter is measured by scintillation spectrometry. Non-specific binding is determined using 1 μmol/L of MDL-7222 (endo-8-methyl-8-azabicyclo [3.2.1]oct-3-yl-3,5-dichlorobenzoate. $IC_{50}$ values are calculated by nonlinear regression analysis. The affinity constants, Ki values, are calculated from the $IC_{50}$ values using the Cheng-Prusoff equation.

Compounds having 5-$HT_3$ receptor antagonist activity which are suitable for use in the invention have an affinity for 5-$HT_3$ receptor (Ki) of not more than about 250 times the Ki of ondansetron for 5-$HT_3$ receptor. This relative activity to ondansetron (Ki of test agent for 5-$HT_3$ receptor / Ki of ondansetron for 5-$HT_3$ receptor), can be determined by assaying the compound of interest and ondansetron using a suitable assay under controlled conditions, for example, conditions which differ primarily in the agent being tested. It is preferred that the relative activity of the 5-$HT_3$ receptor antagonist activity be not more than about 200 times that of ondansetron, for example, not more than about 150 times that of ondansetron, such as not more than about 100 times that of ondansetron, for example, not more than about 50 times that of ondansetron. In a particularly preferred embodiment, the compound having 5-$HT_3$ receptor antagonist activity has a relative activity to ondansetron of not more than about 10.

In certain embodiments, the 5-$HT_3$ receptor antagonist can be selected from indisetron, YM-114 ((R)-2,3-dihydro-1-[(4,5,6,7-tetrahydro-1H-benzimidazol-5-yl-)carbonyl]-1H-indole), granisetron, talipexole, azasetron, bemesetron, tropisetron, ramosetron, ondansetron, palonosetron, lerisetron, alosetron, N-3389, zacopride, cilansetron, E-3620 ([3(S)-endo]-4-amino-5-chloro-N-(8-methyl-8-azabicyclo[3.2.1-]oct-3-yl-2[(1-methyl-2-butynyl)oxy]benzamide), lintopride, KAE-393, itasetron, zatosetron, dolasetron, (±)-zacopride, (+)-renzapride, (-)-YM-060, DAU-6236, EMU-8 and GK-128 [2-[2-methylimidazol-1-yl)methyl]-benzo[*f*]thiochromen-1-one monohydrochloride hemihydrate].

In preferred embodiments, the 5-$HT_3$ receptor antagonist can be selected from indisetron, granisetron, azasetron, bemesetron, tropisetron, ramosetron, ondansetron, palonosetron, lerisetron, alosetron, cilansetron, itasetron, zatosetron, and dolasetron.

The invention relates to a method of treating a functional bowel disorder in a subject in need of treatment. The method comprises administering to a subject in need of treatment a therapeutically effective amount of a compound that has 5-$HT_3$ receptor antagonist activity and NARI activity. The functional bowel disorder can be selected from the group consisting of IBS, functional abdominal bloating, functional constipation and functional diarrhea.

In a particular embodiment, the compounds having 5-$HT_3$ receptor antagonist activity and NARI activity are thieno[2,3-d]pyrimidine derivatives such as those described in U.S. Patent No. 4,695,568, the entire content of which is incorporated herein by reference.

In a specific embodiment, the compounds having 5-$HT_3$ receptor antagonist activity and NARI activity are represented by Formula I:

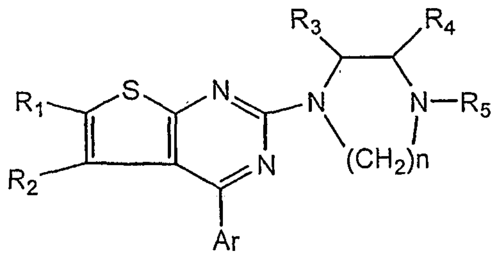

I

wherein, $R_1$ and $R_2$ independently represent hydrogen, halogen or a $C_1$-$C_6$ alkyl group; or $R_1$ and $R_2$ together with the carbon atoms to which they are attached form a cycloalkylene group having 5 to 6 carbon atoms;
$R_3$ and $R_4$ independently represent hydrogen or a $C_1$-$C_6$ alkyl group;
$R_5$ is hydrogen, $C_1$-$C_6$ alkyl,

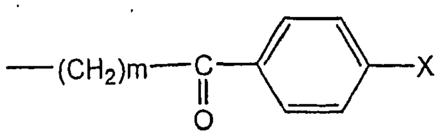 , 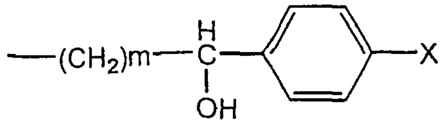

or -C(O)-NH-$R_6$,

wherein m is an integer from about 1 to about 3, X is halogen and $R_6$ is a $C_1$-$C_6$ alkyl group;

Ar is a substituted or unsubstituted phenyl, 2-thienyl or 3-thienyl group; and n is 2 or 3; or a pharmaceutically acceptable salt thereof.

Substituted phenyl, 2-thienyl or 3-thienyl group refers to a phenyl, 2-thienyl or 3-thienyl group in which at least one of the hydrogen atoms available for substitution has been replaced with a group other than hydrogen (i.e., a substituent group). Multiple substituent groups can be present on the phenyl, 2-thienyl or 3-thienyl ring. When multiple substituents are present, the substituents can be the same or different and substitution can be at any of the substitutable sites on the ring. Substituent groups can be, for example, a halogen atom (fluorine, chlorine, bromine or iodine); an alkyl group, for example, a $C_1$-$C_6$ alkyl group such as a methyl, ethyl, propyl, butyl, pentyl or hexyl group; an alkoxy group, for example, a $C_1$-$C_6$ alkoxy group such as methoxy, ethoxy, propoxy, butoxy; a hydroxy group; a nitro group; an amino group; a cyano group; or an alkyl substituted amino group such as methylamino, ethylamino, dimethylamino or diethylamino group.

$C_1$-$C_6$ alkyl group refers to a straight-chain or branched alkyl group having from one to six carbon atoms. For example, the $C_1$-$C_6$ alkyl group can be a strain-chain alkyl such as methyl, ethyl, propyl, etc. Alternatively, the alkyl group can be branched for example, an isopropyl or t-butyl group.

Halogen refers to fluorine, chlorine, bromine or iodine.

In a particular embodiment, the compounds having 5-$HT_3$ receptor antagonist activity and NARI activity are represented by Formula I, wherein $R_1$ is a $C_1$-$C_6$ alkyl group and Ar is a substituted phenyl. In this embodiment, it is preferred that the phenyl group is substituted with a halogen.

In a particularly preferred embodiment, the compounds having 5-$HT_3$ receptor antagonist activity and NARI activity are represented by Formula I, wherein n is 2, $R_1$ is a $C_1$-$C_6$ alkyl group and Ar is a substituted phenyl. Preferably, the phenyl group is substituted with a halogen and the alkyl group of $R_1$ is a methyl group.

In yet another embodiment, the compounds having 5-$HT_3$ receptor antagonist activity and NARI activity are represented by Formula I, wherein $R_1$ is a $C_1$-$C_6$ alkyl group or a halogen and Ar is an unsubstituted phenyl. Further, when $R_1$ is an alkyl group and Ar is an unsubstituted phenyl, $R_2$ can also be a hydrogen or a $C_1$-$C_6$ alkyl group.

In a particularly preferred embodiment, the compounds having 5-$HT_3$ receptor antagonist activity and NARI activity are represented by Formula I, wherein n is 2, $R_1$ is a $C_1$-$C_6$ alkyl group and Ar is an unsubstituted phenyl. In a specific embodiment, wherein n is 2, $R_1$ is a $C_1$-$C_6$ alkyl group and Ar is an unsubstituted phenyl, $R_2$ can be hydrogen or a $C_1$-$C_6$ alkyl group.

In a particularly preferred embodiment, the compound having 5-$HT_3$ receptor antagonist activity and NARI activity is represented by structural Formula II:

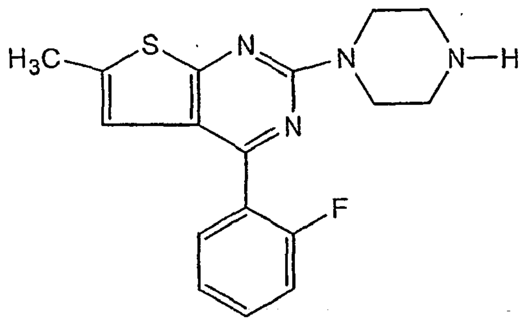

II

or a pharmaceutically acceptable salt thereof. This compound is commonly referred to in the art as MCI-225, also referred to as DDP-225. The chemical name of the structure set forth in the formula is: 4-(2-fluorophenyl)-6-methyl-2-(1-piperazinyl)thieno[2,3-d]pyrimidine.

In a certain embodiment, the functional bowel disorder is IBS. In a particular embodiment, the IBS is diarrhea predominant IBS. In another embodiment, the IBS is alternating constipation/diarrhea IBS. In a further embodiment, the IBS is nonconstipated IBS.

In another embodiment, the method further comprises administering a therapeutically effective amount of an (i.e., one or more) additional therapeutic agent.

Compounds having 5-$HT_3$ receptor antagonist activity and NARI activity, such as the compounds represented by structural Formulas I and II are useful for treating functional bowel disorders such as IBS by virtue of the dual therapeutic modes of action which they can exhibit. That is, the ability to modulate the function of both the 5-$HT_3$ receptor and the noradrenaline reuptake mechanism can provide an enhanced treatment regimen for the subject undergoing treatment.

In a preferred embodiment, compounds having 5-$HT_3$ receptor antagonist activity and NARI activity, such as the compounds of Formula I and II possess one or more characteristics selected from the group consisting of:

a) the substantial absence of anticholinergic effects;
b) the selective inhibition of noradrenaline reuptake as compared to inhibition of serotonin reuptake; and
c) the selective inhibition of noradrenaline reuptake as compared to inhibition of dopamine reuptake.

For example, the specific compound MCI -225 has been shown to be a selective NARI and a 5-$HT_3$ receptor antagonist with substantially no anticholinergic activity. Eguchi et al., Arzneim.-Forschung/Drug Res., 47(12): 1337-47 (1997), reported inhibition constants for MCI-225 for the uptake the [$^3$H]monoamine neurotransmitters noradrenaline, serotonin and dopamine in various rat brain tissues. More specifically, MCI-225 inhibited the uptake of [$^3$H]NA and [$^3$H] 5-HT by synaptosomes from rat hypothalamic tissue with inhibition constants of Ki=35.0 nmol/L and Ki=491 nmol/L, respectively. In addition, MCI-225 inhibited the uptake of [$^3$H]NA and [$^3$H]5-HT by synaptosomes from rat cortical tissue with inhibition constants of Ki=0.696 nmol/L and Ki=1070 nmol/L, respectively. MCI-225 was also reported to inhibit the uptake of serotonin by synaptosomes from rat hippocampal tissue with an inhibition constant of Ki=244 nmol/L. Further, the MCI-225 inhibition constant for the uptake of [$^3$H]DA by synaptosomes from rat striatal tissue was reported as Ki=14,800. MCI-225 did not inhibit Monoamine Oxidase-A (MAO-A) and Monoamine Oxidase-B (MAO-B) activities.

With regard to 5-$HT_3$ receptor antagonist activity, Eguchi *et al.* reported that MCI-225 showed high affinity for the 5-$HT_3$ receptor (Ki less than 100 nmol/L) in comparison to the other receptors tested. In addition, MCI-225 showed affinity for the 5-$HT_3$ receptor similar to that reported for ondansetron in the same radioligand binding assay. Briefly, the inhibition of radiolabeled ligand binding by MCI-225, using a suitable radioligand and tissue combination for the receptor of interest was determined. The receptors tested included, $\alpha_1$, $\alpha_2$, $\beta_1$, $\beta_2$, 5-$HT_1$, 5-$HT_{1A}$, 5-$HT_{1c}$, 5-$HT_2$, 5-$HT_3$, 5-$HT_4$, 5-$HT_6$, 5-$HT_7$, $D_1$, $D_2$, Muscarinic, $M_1$, $M_2$, $M_3$, Nicotonic, $H_1$, $H_2$, GABA-A, GABA-B, BZP, Opiate non-selective, Opiate $\kappa$, Opiate $\mu$, Opiate $\delta$, CRF (Corticotropin Releasing Factor) and glucocorticoid. The $IC_{50}$ values determined for MCI-225, for these additional receptors were all greater than 1 $\mu$mol/L.

The invention further relates to a method of treating a functional bowel disorder in a subject in need thereof, comprising coadministering to said subject a therapeutically effective amount of a 5-$HT_3$ receptor antagonist and a therapeutically effective amount of a NARI. The functional bowel disorder can be selected from IBS, functional abdominal bloating, functional constipation and functional diarrhea.

The invention further relates to a method of treating a functional bowel disorder in a subject in need thereof,

comprising coadministering to said subject a first amount of a 5-$HT_3$ receptor antagonist and a second amount of a NARI, wherein the first and second amounts together comprise a therapeutically effective amount. The functional bowel disorder can be selected from IBS, functional abdominal bloating, functional constipation and functional diarrhea.

In a specific embodiment, the coadministration methods can be used to treat IBS. In a particular embodiment, the IBS is diarrhea predominant IBS. In another embodiment, the IBS is alternating constipation/diarrhea IBS. In a further embodiment, the IBS is nonconstipated IBS.

In another embodiment, the coadministration methods further comprise administering a therapeutically effective amount of an (i.e., one or more) additional therapeutic agent.

In certain embodiments of the coadministration method, the 5-$HT_3$ receptor antagonist can be selected from indisetron, YM-114 ((R)-2,3-dihydro-1-[(4,5,6,7-tetrahydro-1H-benzimidazol-5-yl-)carbonyl]-1H-indole), granisetron, talipexole, azasetron, bemesetron, tropisetron, ramosetron, ondansetron, palonosetron, lerisetron, alosetron, N-3389, zacopride, cilansetron, E-3620 ([3(S)-endo]-4-amino-5-chloro-N-(8-methyl-8-azabicyclo[3.2.1-]oct-3-yl-2[(1-methyl-2-butynyl)oxy]benzamide), lintopride, KAE-393, itasetron, zatosetron, dolasetron, (±)-zacopride, (±)-renzapride, (-)-YM-060, DAU-6236, BIMU-8 and GK-128 [2-[2-methylimidazol-1-yl)methyl]-benzo[*f*]thiochromen-1-one monohydrochloride hemihydrate].

In preferred embodiments, the 5-$HT_3$ receptor antagonist can be selected from indisetron, granisetron, azasetron, bemesetron, tropisetron, ramosetron, ondansetron, palonosetron, lerisetron, alosetron, cilansetron, itasetron, zatosetron, and dolasetron.

In certain embodiments, the NARI compound can be selected from venlafaxine, duloxetine, buproprion, milnacipran, reboxetine, lefepramine, desipramine, nortriptyline, tomoxetine, maprotiline, oxaprotiline, levoprotiline, viloxazine and atomoxetine.

In a preferred embodiment, the NARI compound can be selected from reboxetine, lefepramine, desipramine, nortriptyline, tomoxetine, maprotiline, oxaprotiline, levoprotiline, viloxazine and atomoxetine.

In a preferred embodiment, the NARI compound possesses one or more characteristics selected from the group consisting of:

a) the substantial absence of anticholinergic effects;
b) the selective inhibition of noradrenaline reuptake as compared to inhibition of serotonin reuptake; and
c) the selective inhibition of noradrenaline reuptake as compared to inhibition of dopamine reuptake.

In addition, the invention relates to a method of treating a functional bowel disorder in a subject in need thereof comprising administering a therapeutically effective amount of a-NARI. In this embodiment, the NARI is characterized by the substantial absence of anticholinergic effects.

In a further embodiment, the NARI possesses selective inhibition of noradrenaline reuptake as compared to inhibition of serotonin reuptake and/or selective inhbition of noradrenaline reuptake as compared to inhibition of dopamine reuptake. The functional bowel disorder can be selected from IBS, functional abdominal bloating, functional constipation and functional diarrhea.

In a specific embodiment, the administration of the NARI can be used to treat IBS. In a particular embodiment, the IBS is diarrhea predominant IBS. In another embodiment, the IBS is alternating constipation/diarrhea IBS. In a further embodiment, the IBS is nonconstipated IBS.

In another embodiment, the method further comprises administering a therapeutically effective amount of an (i.e., one or more) additional therapeutic agent.

The invention further relates to pharmaceutical compositions useful for the treatment of a functional bowel disorder. The pharmaceutical composition comprises a first amount of a 5-$HT_3$ receptor antagonist compound and a second amount of a NARI compound. The pharmaceutical compositions of the present invention can optionally contain a pharmaceutically acceptable carrier. The 5-$HT_3$ receptor antagonist and the NARI can each be present in the pharmaceutical composition in a therapeutically effective amount. In another aspect, said first and second amounts can together comprise a therapeutically effective amount.

In a further embodiment, the pharmaceutical composition further comprises an (i.e., one or more) additional therapeutic agent.

The pharmaceutical composition can be used in the treatment of a functional bowel disorder in a subject in need of treatment. As such, the invention relates to a method of treating a functional bowel disorder in a subject in need of treatment comprising administering to the subject a therapeutically effective amount of a pharmaceutical composition as described herein. The functional bowel disorder can be selected from IBS, functional abdominal bloating, functional constipation and functional diarrhea. In a certain embodiment, the functional bowel disorder is IBS. In a particular embodiment, the IBS is diarrhea predominant IBS. In another embodiment, the IBS is alternating constipation/diarrhea IBS. In a further embodiment, the IBS is nonconstipated IBS.

An additional therapeutic agent suitable for use in the methods and pharmaceutical compositions described

herein, can be, but is not limited to, for example: an antispasmodic agent, such as an anticholinergic drug (e.g., dicyclomine, hyoscyamine and cimetropium); a smooth muscle relaxant (e.g., mebeverine); a calcium blocker (e.g., verapamil, nifedipine, octylonium bromide, peppermint oil and pinaverium bromide); an antidiarrheal agent (e.g., loperamide and dipehnoxylate); a stool bulking agent (e.g., psyllium, polycarbophil); an antiafferent agent (e.g., octreotide and fedotozine); a prokinetic agent, such as a dopamine antagonist (e.g., domperidone and metoclopramide) or a 5-$HT_4$ antagonist (e.g., cisapride); a psychotropic agent, such as a tricyclic antidepressant; or any combination thereof.

FUNCTIONAL BOWEL DISORDERS

Functional Bowel Disorders (FBDs) are functional gastrointestinal disorders having symptoms attributable to the mid or lower gastrointestinal tract. FBDs can include, Irritable Bowel Syndrome (IBS), functional abdominal bloating, functional constipation and functional diarrhea (see, for example, Thompson et al., Gut, 45 (Suppl II):II43-II47 (1999)).

IBS

IBS comprises a group of functional bowel disorders in which abdominal discomfort or pain is associated with defecation or change in bowel habit and with features of disordered defecation. Diagnostic criteria for IBS are at least 12 weeks, which need not be consecutive, in the preceding 12 months of abdominal discomfort or pain that has two of three features:

a) Relieved with defecation; and/or
b) Onset associated with a change in frequency of stool; and/or
c) Onset associated with a change in form (appearance) of stool.

The following symptoms cumulatively support the diagnosis of IBS:

- abnormal stool frequency (for research purposes "abnormal" can be defined as 3/day and <3/week);
- abnormal stool form (lumpy/hard or loose/watery stool);
- abnormal stool passage (straining, urgency, or feeling of incomplete evacuation);
- passage of mucus;
- bloating or feeling of abdominal distension.

Further, subjects with IBS exhibit visceral hypersensitivity, the presence of which physiological studies have shown is the most consistent abnormality in IBS.

It is believed that the pain associated with IBS is primarily a result of this hypersensitivity of the visceral afferent nervous system. For example, patients and controls were evaluated for their pain thresholds in response to progressive distension of the sigmoid colon induced by a balloon. At the same volume of distension, the patients reported higher pain scores compared to controls. This finding has been reproduced in many studies and with the introduction of the barostat, a computerized distension device, the distension procedures have been standardized. Two concepts of visceral hypersensitivity, hyperalgesia and allodynia, have been introduced. More specifically, hyperalgesia refers to the situation in which normal visceral sensations are experienced at lower intraluminal volumes. While for a finding of allodynia, pain or discomfort is experienced at volumes usually producing normal internal sensations (see, for example, Mayer E.A. and Gebhart, G.F., Basic and Clinical Aspects of Chronic Abdominal Pain, Vol 9, 1st ed. Amsterdam: Elsevier, 1993:3-28).

As such, IBS is a functional bowel disorder in which abdominal pain or discomfort is associated with defecation or a change in bowel habit. Therefore, IBS has elements of an intestinal motility disorder, a visceral sensation disorder, and a central nervous disorder. While the symptoms of IBS have a physiological basis, no physiological mechanism unique to IBS has been identified. In some cases, the same mechanisms that cause occasional abdominal discomfort in healthy individuals operate to produce the symptoms of IBS. The symptoms of IBS are therefore a product of quantitative differences in the motor reactivity of the intestinal tract, and increased sensitivity to stimuli or spontaneous contractions.

FUNCTIONAL ABDOMINAL BLOATING

Functional abdominal bloating comprises a group of functional bowel disorders which are dominated by a feeling of abdominal fullness or bloating and without sufficient criteria for another functional gastrointestinal disorder.

Diagnostic criteria for functional abdominal bloating are at least 12 weeks, which need not be consecutive, in the preceding 12 months of:

(1) Feeling of abdominal fullness, bloating or visible distension; and

(2) Insufficient criteria for a diagnosis of functional dyspepsia, IBS, or other functional disorder.

FUNCTIONAL CONSTIPATION:

Functional constipation comprises a group of functional disorders which present as persistent difficult, infrequent or seemingly incomplete defecation.

The diagnostic criteria for functional constipation are at least 12 weeks, which need not be consecutive, in the preceding 12 months of two or more of:

(1) Straining in 1/4 defecations;
(2) Lumpy or hard stools in >1/4 defecations;
(3) Sensation of incomplete evacuation in >1/4 defecations;
(4) Sensation of anorectal obstruction/blockade in >1/4 defecation;
(5) Manual maneuvers to faciltate >1/4 defecations (e.g., digital evacuation, support of the pelvic floor); and/or
(6) <3 defecations/week.

Loose stools are not present, and there are insufficient criteria for IBS.

FUNCTIONAL DIARRHEA

Functional diarrhea is continuous or recurrent passage of loose (mushy) or watery stools without abdominal pain. The diagnostic criteria for functional diarrhea are at least 12 weeks, which need not be consecutive, in the preceding 12 months of:

(1) Liquid (mushy) or watery stools;
(2) Present >3/4 of the time; and
(3) No abdominal pain.

Subject, as used herein, refers to animals such as mammals, including, but not limited to, primates (e.g., humans), cows, sheep, goats, horses, pigs, dogs, cats, rabbits, guinea pigs, rats, mice or other bovine, ovine, equine, canine, feline, rodent or murine species.

As used herein, therapeutically effective amount refers to an amount sufficient to elicit the desired biological response. In the present invention the desired biological response is a reduction (complete or partial) of at least one symptom associated with the functional bowel disorder being treated. For example, when the functional bowel disorder is IBS, a reduction in the pain or discomfort associated with IBS is considered a desired biological response. As with any treatment, particularly treatment of a multi-symptom disorder, for example, IBS, it is advantageous to treat as many disorder-related symptoms which the subject experiences. As such, when the subject is being treated for IBS a reduction in the pain or discomfort associated with IBS and a reduction in at least one other symptom of IBS selected from abnormal stool frequency, abnormal stool form, abnormal stool passage, passage of mucus and bloating or feeling of abdominal distension is preferred.

MODES OF ADMINISTRATION

The compounds for use in the method of the invention can be formulated for oral, transdermal, sublingual, buccal, parenteral, rectal, intranasal, intrabronchial or intrapulmonary administration. For oral administration the compounds can be of the form of tablets or capsules prepared by conventional means with pharmaceutically acceptable excipients such as binding agents (e.g., polyvinylpyrrolidone, hydroxypropylcellulose or hydroxypropylmethylcellulose); fillers (e.g., cornstarch, lactose, microcrystalline cellulose or calcium phosphate); lubricants (e.g., magnesium stearate, talc, or silica); disintegrates (e.g., sodium starch glycollate); or wetting agents (e.g., sodium lauryl sulphate). If desired, the tablets can be coated using suitable methods and coating materials such as OPADRY® film coating systems available from Colorcon, West Point, PA (e.g., OPADR® OY Type, OY-C Type, Organic Enteric OY-P Type, Aqueous Enteric OY-A Type, OY-PM Type and OPADRY® White , 32K18400). Liquid preparation for oral administration can be in the form of solutions, syrups or suspensions. The liquid preparations can be prepared by conventional means with pharmaceutically acceptable additives such as suspending agents (e.g., sorbitol syrup, methyl cellulose or hydrogenated edible fats); emulsifying agent (e.g., lecithin or acacia); non-aqueous vehicles (e.g., almond oil, oily esters or ethyl alcohol); and preservatives (e.g., methyl or propyl p-hydroxy benzoates or sorbic acid).

For buccal administration, the compounds for use in the method of the invention can be in the form of tablets or lozenges formulated in a conventional manner.

For parenteral admininstration, the compounds for use in the method of the invention can be formulated for injection or infusion, for example, intravenous, intramuscular or subcutaneous injection or infusion, or for administration in a bolus dose and/or continuous infusion. Suspensions, solutions or emulsions in an oily or aqueous vehicle, optionally containing other formulatory agents such as suspending, stabilizing and/or dispersing agents can be used.

For rectal administration, the compounds for use in the method of the invention can be in the form of suppositories.

For sublingual administration, tablets can be formulated in conventional manner.

For intranasal, intrabronchial or intrapulmonary administration, conventional formulations can be employed.

Further, the compounds for use in the method of the invention can be formulated in a sustained release preparation. For example, the compounds can be formulated with a suitable polymer or hydrophobic material which provides sustained and/or controlled release properties to the active agent compound. As such, the compounds for use the method of the invention can be administered in the form of microparticles for example, by injection or in the form of wafers or discs by implantation.

Additional dosage forms of this invention include dosage forms as described in U.S. Pat. No. 6,340,475, U.S. Pat. No. 6,488,962, U.S. Pat. No. 6,451,808, U.S. Pat. No. 6,340,475, U.S. Pat. No. 5,972,389, U.S. Pat. No. 5,582,837, and U.S. Pat. No. 5,007,790. Additional dosage forms of this invention also include dosage forms as described in U.S. Pat. Application No. 20030147952, U.S. Pat. Application No. 20030104062, U.S. Pat. Application No. 20030104053, U.S. Pat. Application No. 20030044466, U.S. Pat. Application No. 20030039688, and U.S. Pat. Application No. 20020051820. Additional dosage forms of this invention also include dosage forms as described in PCT Patent Application WO 03/35041, PCT Patent Application WO 03/35040, PCT Patent Application WO 03/35029, PCT Patent Application WO 03/35177, PCT Patent Application WO 03/35039, PCT Patent Application WO 02/96404, PCT Patent Application WO 02/32416, PCT Patent Application WO 01/97783, PCT Patent Application WO 01/56544, PCT Patent Application WO 01/32217, PCT Patent Application WO 98/55107, PCT Patent Application WO 98/11879, PCT Patent Application WO 97/47285, PCT Patent Application WO 93/18755, and PCT Patent Application WO 90/11757.

In one embodiment, the dosage forms of the present invention include pharmaceutical tablets for oral administration as described in U.S. Patent Application No. 20030104053. For example, suitable dosage forms of the present invention can combine both immediate-release and prolonged-release modes of drug delivery. The dosage forms of this invention include dosage forms in which the same drug is used in both the immediate-release and the prolonged-release portions as well as those in which one drug is formulated for immediate release and another drug, different from the first, is formulated for prolonged release. This invention encompasses dosage forms in which the immediate-release drug is at most sparingly soluble in water, i.e., either sparingly soluble or insoluble in water, while the prolonged-release drug can be of any level of solubility.

More particularly, in a further embodiment, the prolonged-release portion of the dosage form can be a dosage form that delivers its drug to the digestive system continuously over a period of time of at least an hour and preferably several hours and the drug is formulated as described in in U.S. Patent Application No. 20030104053. In said embodiment, the immediate-release portion of the dosage form can be a coating applied or deposited over the entire surface of a unitary prolonged-release core, or can be a single layer of a tablet constructed in two or more layers, one of the other layers of which is the prolonged-released portion and is formulated as described in U.S. Patent Application No. 20030104053.

In another embodiment of the invention, the supporting matrix in controlled-release tablets or controlled release portions of tablets is a material that swells upon contact with gastric fluid to a size that is large enough to promote retention in the stomach while the subject is in the digestive state, which is also referred to as the postprandial or "fed" mode. This is one of two modes of activity of the stomach that differ by their distinctive patterns of gastroduodenal motor activity. The "fed" mode is induced by food ingestion and begins with a rapid and profound change in the motor pattern of the upper gastrointestinal (GI) tract. The change consists of a reduction in the amplitude of the contractions that the stomach undergoes and a reduction in the pyloric opening to a partially closed state. The result is a sieving process that allows liquids and small particles to pass through the partially open pylorus while indigestible particles that are larger than the pylorus are retropelled and retained in the stomach. This process causes the stomach to retain particles that are greater than about 1 cm in size for about 4 to 6 hours. The controlled-release matrix in these embodiments of the invention is therefore selected as one that swells to a size large enough to be retropelled and thereby retained in the stomach, causing the prolonged release of the drug to occur in the stomach rather than in the intestines. Disclosures of oral dosage forms that swell to sizes that will prolong the residence time in the stomach are found in U.S. Pat. No. 6,448,962, U.S. Pat. No. 6,340,475, U.S. Pat. No. 5,007,790, U.S. Pat. No. 5,582,837, U.S. Pat. No. 5,972,389, PCT Patent Application WO 98/55107, U.S. Patent Application No. 20010018707, U.S. Patent Application No. 20020051820, U.S. Patent Application No. 20030029688, U.S. Patent Application No. 20030044466, U.S. Patent Application No. 20030104062, U.S. Patent Application No. 20030147952, U.S. Patent Application No. 20030104053, and PCT Patent Application WO 96/26718. In particular, gastric retained dosage formulations for specific drugs have also been described, for example, a gastric retained dosage formulation for gabapentin is disclosed in PCT Patent Application WO 03/035040.

COADMINISTRATION

In practicing the methods of the invention, coadministration refers to administration of a first amount of a 5-$HT_3$ receptor antagonist compound and a second amount of a NARI compound to treat a functional bowel disorder, for example IBS. Coadministration encompasses administration of the first and second amounts of the compounds of the coadministration in an essentially simultaneous manner, such as in a single pharmaceutical composition, for example, capsule or tablet having a fixed ratio of first and second amounts, or in multiple, separate capsules or tablets for each. In addition, such coadministration also encompasses use of each compound in a sequential manner in either order. When coadministration involves the separate administration of the NARI and 5-$HT_3$ receptor antagonist, the compounds are administered sufficiently close in time to have the desired therapeutic effect.

DOSING

The therapeutically effective amount or dose of (a) a compound having dual therapeutic modes of action (i.e., 5-$HT_3$ receptor antagonist activity and NARI activity); (b) a 5-$HT_3$ receptor antagonist and NARI in combination; or (c) a NARI alone, will depend on the age, sex and weight of the patient, the current medical condition of the patient and the nature of the functional bowel disorder being treated. The skilled artisan will be able to determine appropriate dosages depending on these and other factors.

As used herein, continuous dosing refers to the chronic administration of a selected active agent.

As used herein, as-needed dosing, also known as "pro re nata" "prn" dosing, and "on demand" dosing or administration is meant the administration of a therapeutically effective dose of the compound(s) at some time prior to commencement of an activity wherein suppression of a functional bowel disorder would be desirable. Administration can be immediately prior to such an activity, including about 0 minutes, about 10 minutes, about 20 minutes, about 30 minutes, about 1 hour, about 2 hours, about 3 hours, about 4 hours, about 5 hours, about 6 hours, about 7 hours, about 8 hours, about 9 hours, or about 10 hours prior to such an activity, depending on the formulation. In a particular embodiment, drug administration or dosing is on an as-needed basis, and does not involve chronic drug administration. With an immediate release dosage form, as-needed administration can involve drug administration immediately prior to commencement of an activity wherein suppression of the symptoms of a functional bowel disorder would be desirable, but will generally be in the range of from about 0 minutes to about 10 hours prior to such an activity, preferably in the range of from about 0 minutes to about 5 hours prior to such an activity, most preferably in the range of from about 0 minutes to about 3 hours prior to such an activity.

For example, a suitable dose of the 5-$HT_3$ receptor antagonist can be in the range of from about 0.001 mg to about 500 mg per day, such as from about 0.01 mg to about 100 mg, for example, from about 0.05 mg to about 50 mg, such as from about 0.5 mg to about 25 mg per day. The dose can be administered in a single dosage or in multiple dosages, for example from 1 to 4 or more times per day. When multiple dosages are used, the amount of each dosage can be the same or different.

For example, a suitable dose of the NARI compound can be in the range of from about 0.001 mg to about 1000 mg per day, such as from about 0.05 mg to about 500 mg, for example, from about 0.03mg to about 300 mg, such as from about 0.02 mg to about 200 mg per day. The dose can be administered in a single dosage or in multiple dosages, for example from 1 to 4 or more times per day. When multiple dosages are used, the amount of each dosage can be the same or different.

For example, a suitable dose of the compound having both 5-$HT_3$ receptor antagonist and NARI activity can be in the range of from about 0.001 mg to about 1000 mg per day, such as from about 0.05 mg to about 500 mg, for example, from about 0.03 mg to about 300 mg, such as from about 0.02 mg to about 200 mg per day. In a particular embodiment, a suitable dose of the compound having both 5-$HT_3$ receptor antagonist and NARI activity can be in the range of from about 0.1 mg to about 50 mg per day, such as from about 0.5 mg to about 10 mg per day, such as about 0.5, 1, 2, 3, 4, 5, 6, 7, 8, 9, or 10 mg per day. The dose per day can be administered in a single dosage or in multiple dosages, for example from 1 to 4 or more times per day. When multiple dosages are used, the amount of each dosage can be the same or different. For example a dose of 1 mg per day can be administered as two 0.5 mg doses, with about a 12 hour interval between doses.

It is understood that the amount of compound dosed per day can be administered every day, every other day, every 2 days, every 3 days, every 4 days, every 5 days, etc. For example, with every other day administration, a 5 mg per day dose can be initiated on Monday with a first subsequent 5 mg per day dose administered on Wednesday, a second subsequent 5 mg per day dose administered on Friday, etc.

The compounds for use in the method of the invention can be formulated in unit dosage form. The term "unit dosage form" refers to physically discrete units suitable as unitary dosage for subjects undergoing treatment, with each unit containing a predetermined quantity of active material calculated to produce the desired therapeutic effect, optionally in association with a suitable pharmaceutical carrier. The unit dosage form can be for a single daily dose or one of

multiple daily doses (e.g., about 1 to 4 or more times per day). When multiple daily doses are used, the unit dosage form can be the same or different for each dose.

For the compounds having both NARI and 5-$HT_3$ receptor antagonist activity, each dosage can typically contain from about 0.001 mg to about 1000 mg, such as from about 0.05 mg to about 500 mg, for example, from about 0.03 mg to about 300 mg, such as about 0.02 mg to about 200 mg of the active ingredient.

When the method of treatment comprises coadministration of a NARI and a 5-$HT_3$ receptor antagonist each dose can typically contain from about .001 mg to about 1000 mg, such as from about 0.05 mg to about 500 mg, for example, from about 0.03 mg to about 300 mg, such as about 0.02 mg to about to about 200 mg of the NARI and typically can contain from about 0.001 mg to about 500 mg, such as from about 0.01 mg to about 100 mg, for example, from about 0.05 mg to about 50 mg, such as about 0.5 mg to about 25 mg of the 5-$HT_3$ receptor antagonist.

When the method of treatment comprises administration of a NARI alone, each dose can typically contain from about .001 mg to about 1000 mg, such as from about 0.05 mg to about 500 mg, for example, from about 0.03 mg to about 300 mg, such as 0.02 to about to about 200 mg of the active ingredient.

The invention further includes a kit for treating a functional bowel disorder. The kit comprises at least one compound having both 5-$HT_3$ receptor antagonist activity and NARI activity (e.g., a single compound) and an instruction insert for administering the compound according to the method of the invention. In addition, the kit can comprise a first compound which is a 5-$HT_3$ receptor antagonist and a second compound which is a NARI and an instruction insert for administering the compounds according to the method of the invention. The first and second compounds can be in separate dosage forms or combined in a single dosage form.

As used herein, the term pharmaceutically acceptable salt refers to a salt of the administered compounds prepared from pharmaceutically acceptable non-toxic acids including inorganic acids, organic acids, solvates, hydrates, or clathrates thereof. Examples of such inorganic acids are hydrochloric, hydrobromic, hydroiodic, nitric, sulfuric, and phosphoric. Appropriate organic acids may be selected, for example, from aliphatic, aromatic, carboxylic and sulfonic classes of organic acids, examples of which are formic, acetic, propionic, succinic, camphorsulfonic, citric, fumaric, gluconic, isethionic, lactic, malic, mucic, tartaric, para-toluenesulfonic, glycolic, glucuronic, maleic, furoic, glutamic, benzoic, anthranilic, salicylic, phenylacetic, mandelic, embonic (pamoic), methanesulfonic, ethanesulfonic, pantothenic, benzenesulfonic (besylate), stearic, sulfanilic, alginic, galacturonic, and the like.

It is understood that 5-$HT_3$ receptor antagonists, NARIs and single compounds having both NARI and 5-$HT_3$ antagonist activities can be identified, for example, by screening libraries or collections of molecules using suitable methods. Another source for the compounds of interest are combinatorial libraries which can comprise many structurally distinct molecular species. Combinatorial libraries can be used to identify lead compounds or to optimize a previously identified lead. Such libraries can be manufactured by well-known methods of combinatorial chemistry and screened by suitable methods.

The invention also relates to a method of processing a claim under a health insurance policy submitted by a claimant seeking reimbursement for costs associated with the treatment of a functional bowel disorder, as described herein.

In one embodiment, the method of processing a claim under a health insurance policy submitted by a claimant seeking reimbursement for costs associated with treatment of a functional bowel disorder, wherein said treatment comprises coadministering to a subject a first amount of a 5-$HT_3$ receptor antagonist and a second amount of a noradrenaline reuptake inhibitor, wherein the first and second amounts together comprise a therapeutically effective amount comprising: reviewing said claim; determining whether said treatment is reimbursable under said insurance policy; and processing said claim to provide partial or complete reimbursement of said costs.

In one embodiment, the functional bowel disorder being treated is irritable bowel syndrome.

In a particular embodiment, the irritable bowel syndrome is diarrhea predominant irritable bowel syndrome.

In a further embodiment, the irritable bowel syndrome is alternating constipation/diarrhea irritable bowel syndrome.

The invention also relates to a method for processing a claim under a health insurance policy submitted by a claimant seeking reimbursement for costs associated with treatment of a functional bowel disorder, wherein said treatment comprises coadministering to a subject a therapeutically effective amount of a 5-$HT_3$ receptor antagonist and a therapeutically effective amount of a noradrenaline reuptake inhibitor comprising: reviewing said claim; determining whether said treatment is reimbursable under said insurance policy; and processing said claim to provide partial or complete reimbursement of said costs.

In one embodiment, the functional bowel disorder being treated is irritable bowel syndrome.

In a particular embodiment, the irritable bowel syndrome is diarrhea predominant irritable bowel syndrome.

In a further embodiment, the irritable bowel syndrome is alternating constipation/diarrhea irritable bowel syndrome.

The invention also relates to a method for processing a claim under a health insurance policy submitted by a claimant seeking reimbursement for costs associated with treatment of a functional bowel disorder, wherein said treatment

comprises administering to a subject a therapeutically effective amount of a compound having 5-$HT_3$ receptor antagonist activity and noradrenaline reuptake inhibitor activity comprising: reviewing said claim; determining whether said treatment is reimbursable under said insurance policy; and processing said claim to provide partial or complete reimbursement of said costs.

In a particular embodiment, the compound having 5-$HT_3$ receptor antagonist activity and noradrenaline reuptake inhibitor activity is MCI-225.

In one embodiment, the functional bowel disorder being treated is irritable bowel syndrome.

In a particular embodiment, the irritable bowel syndrome is diarrhea predominant irritable bowel syndrome.

In a further embodiment, the irritable bowel syndrome is alternating constipation/diarrhea irritable bowel syndrome.

The invention further relates to a method for processing a claim under a health insurance policy submitted by a claimant seeking reimbursement for costs associated with treatment of a functional bowel disorder, wherein said treatment comprises administering to a subject a therapeutically effective amount of a noradrenaline reuptake inhibitor, wherein the noradrenaline reuptake inhibitor characterized by the substantial absence of anticholinergic effects comprising: reviewing said claim; determining whether said treatment is reimbursable under said insurance policy; and processing said claim to provide partial or complete reimbursement of said costs.

In one embodiment, the functional bowel disorder being treated is irritable bowel syndrome.

In a particular embodiment, the irritable bowel syndrome is diarrhea predominant irritable bowel syndrome.

In a further embodiment, the irritable bowel syndrome is alternating constipation/diarrhea irritable bowel syndrome.

PHARMACOLOGICAL METHODS

DISTENSION MODELS

A variety of assays can be used to assess visceromotor and pain responses to rectal distension. See, for example, Gunter et al., Physiol. Behav., 69(3): 379-82 (2000), Depoortere et al., J. Pharmacol. and Exp. Ther., 294(3): 983-990 (2000), Morteau et al., Fund. Clin. Pharmacol., 8(6): 553-62 (1994), Gibson et al., Gastroenterology (Suppl. 1), 120(5): A19-A20 (2001) and Gschossmann et al., Eur. J. Gastro. Hepat., 14(10): 1067-72 (2002) the entire contents of which are each incorporated herein by reference.

VISCERAL PAIN

Visceral pain can lead to visceral reactions which can manifest themselves as, for example, contractions of the abdominal muscles. The number of contractions of the abdominal muscles occurring after a mechanical pain stimulus produced by distending the large intestine can thus be a measurement for determining visceral sensitivity to pain.

The inhibiting action of a test agent on distension-induced contractions can be tested in rats. The distension of the large intestine with an introduced balloon can be used as the stimulus; the contraction of the abdominal muscles can be measured as the response.

For example, one hour after sensitizing of the large intestine by instillation of a weak acetic acid solution, a latex balloon is introduced and inflated sequentially in a stepwise fashion to about 50-100 mbar for about 5-10 minutes. Pressure values can also be expressed as cm $H_2O$ at 4°C (mbar X 1.01973 = cm $H_20$ at 4°C). During this time, the contractions of the abdominal muscles are counted. About 20 minutes after subcutaneous administration of the test agent, this measurement is repeated. The action of the test agent is calculated as a percentage reduction in the counted contractions compared with the control (i.e., non-sensitized rats).

GASTROINTESTINAL (GI) MOTILITY MODEL

The investigation of gastrointestinal motility can be based on either the in vivo recording of mechanical or electrical events associated intestinal muscle contractions in whole animals or the activity of isolated gastrointestinal intestinal muscle preparations recorded in vitro in organ baths (see, for example, Yaun et al., Br. J. Pharmacol., 112(4): 1095-1100(1994), Jin et al., J. Pharm. Exp. Ther., 288(1): 93-97 (1999) and Venkova et al., J. Pharm. Exp. Ther., 300 (3):1046-1052 (2002)). The in vivo recordings, especially in conscious freely moving animals, have the advantage of characterizing motility patterns and propulsive activity that are directly relevant to the motor function of the GI tract. In comparison, in vitro studies provide data about the mechanisms and site of action of agents directly affecting contractile activity and are a classic tool to distinguish between effects on the circular and /or longitudinal intestinal smooth muscle layers.

IN VIVO

COLONIC CONTRACTILITY

Ambulatory telemetric motility recordings provide a suitable way to investigate intestinal motility in conscious animals during long-lasting time periods. Telemetric recording of colonic motility has been introduced to study propagating contractile activity in the unprepared colon of conscious freely moving animals. Yucatan mini-pigs, present an excellent animal model for motility investigations, based on the anatomical and functional similarities between the gastrointestinal tract in the human and the mini-pig. To be prepared for studies of colonic motility, young mini-pigs undergo a surgical procedure to establish a permanent chronic cecal fistula.

During an experimental trial, the animals are housed in an animal facility under controlled conditions and receive a standard diet with water available ad libitum. Telemetric recording of colonic motility in a segment of proximal colon in the mini-pig is carried out for approximately one week (McRorie et al., Dig. Dis. Sci. 43: 957-963 (1998); Kuge et al., Dig. Dis. Sci. 47: 2651-6 (2002)). The data obtained in each recording session can be used to define the mean amplitude and the total number of propagating contractions, the number of high and low velocity propagating contractions, the number of long and short duration propagating contractions and to estimate the relative shares of each type contractions as % of total contractile activity. A summarized motility index (MI), characterizing colonic contractile activity, can be calculated using the following equation:

$$MI = \frac{\text{\# of contractions/24 hr. x area under the pressure peak}}{\text{24 hr.}}$$

COLONIC MOTILITY

Female rats are administered, TNBS in ethanol or saline (control), intracolonically. The catheter tip is positioned between 2 and 6 cm from the anal verge (n=6/group). Three days following TNBS administration, the animals are food restricted overnight and on the following morning are anesthetized with urethane and are instrumented for physiological/pharmacological experimentation.

A ventral incision is made on the ventral surface of the neck, a jugular catheter is inserted and secured with ligatures, and the skin wound is closed with suture. An intra-colonic balloon-tipped catheter fashioned from condom reservoir tip and tubing is inserted anally and positioned with the balloon at approximately 4 cm from the anal verge. Connection via 3-way stopcock to a syringe pump and pressure transducer allows for simultaneous balloon volume adjustment and pressure recording. Fine wire electrodes are inserted into the external anal sphincter (EAS) and the abdominal wall musculature to permit electromyographic (EMG) recording. With this preparation, intra-colonic pressure, colonic motility, colonic sensory thresholds via abdominal EMG firing, and EAS firing frequency and amplitude is quantified in both control and irritated animals.

Following a control period of about 1 hour at a balloon volume of about 0.025 ml to establish baseline colonic motility and associated non-noxious viscero-somatic reflex measurements, three consecutive escalating ramps of stepwise or continuous balloon inflation are conducted. Following the completion of each volume ramp, the balloons are deflated for 30 minutes for recovery and collection of additional colonic motility measurements. EMG and colonic pressure responses to balloon inflation are measured and analyzed as sensitivities to colorectal distension (CRD). Administration of pharmacological agents is conducted in an escalating dose-response protocol and begins following the last control CRD balloon deflation.

IN VITRO

Recordings of contractile activity of isolated smooth muscle preparations can be used to study selected aspects of muscle function under conditions where the influence of "external" factors (circulating hormones etc.) is removed, while the muscle itself retains its in vivo capacity.

Studies are performed using smooth muscle strips (or whole intestinal segments) mounted vertically in organ baths with one end fixed and the other attached to isometric force transducers. The muscles are continuously bathed in modified Krebs bicarbonate buffer, maintained at 37°C and aerated with 95% $O_2$ and 5% $CO_2$, The tissues are allowed to equilibrate at initial length (Li- at which tension is zero) for approximately 5 minutes, and then are gradually stretched by small force increments to optimal length (Lo - the length at which maximal active tension is generated in response to an agonist). Experiments should be performed at Lo to provide standardized spontaneous activity and pharmacological

responses. The most commonly used recording procedures involve isometric transducers attached to an appropriate recording device. Mechanical responses to stimulation of enteric nerve terminals can be studied in organ baths supplied with pairs of platinum electrodes connected to a physiological electrical stimulator. Isolated smooth muscle preparations can be used also to study length-tension relationships, which provide characteristics of the active and passive properties of the smooth muscle.

CLINICAL EVALUATION

TRIAL DESIGN FOR A PHASE II

The phase II is a dose ranging study that is randomized, double blind placebo controlled parallel group multicenter study in adult (age 18 and over) men and women. In some studies, the patient population can be limited to women.

This is a 2-week run in study with a 4 or 12-week active treatment phase followed by a 2-week minimum follow-up phase to assess treatment of drug in patients with IBS. Subjects will need to fulfill Rome II-type criteria for IBS with at least 6 months of symptoms. Subjects are ambulatory outpatients, have evidence of a recent examination of the large intestine, with no evidence of other serious medical conditions including inflammatory bowel disease.

There are three phases to the study. There is a 2-week screening period to confirm the symptomatology and record changes in bowel habit. Randomization of all subjects that continue to be eligible will be made after that 2-week period to a group. Subjects are assigned to a treatment group (either one of the active groups or placebo) and continuously receive study drug for a 4 or 12-week period. Subjects continue, as they did during the screening period, to record abdominal pain/discomfort and other lower GI symptoms throughout the 4 or 12-week period. Following completion of the treatment period subjects continue to be record symptoms during a 2-week minimum follow-up period with on-going monitoring.

Endpoints include measurement of adequate relief of abdominal pain/discomfort, a comparison of the proportion of pain/discomfort-free days during the treatment period, change in stool consistency, change in stool frequency and change in gastrointestinal transit.

EXEMPLIFICATION

The present invention will now be illustrated by the following Examples, which are not intended to be limiting in any way.

EXAMPLE 1: EVALUATION OF MCI-225 IN A MODEL OF VISCEROMOTOR RESPONSE TO COLORECTAL DISTENSION

TREATMENT OF IBS USING MCI-225

The ability of MCI-225 to reverse acetic acid-induced colonic hypersensitivity in a rodent model of irritable bowel syndrome was assessed. Specifically, the experiments described herein investigated the effect of MGI-225 on visceromotor responses in a rat model of acetic acid-induced colonic hypersensitivity in the distal colon of non-stressed rats.

METHOD

Animals

Adult male Fisher rats were housed (2 per cage) in the animal facility at standard conditions. Following one week of acclimatization to the animal facility, the rats were brought to the laboratory and handled daily for another week to get used to the environment and the research associate performing the experiments.

Visceromotor Responses to Colorectal Distension (CRD)

The visceromotor behavioral response to colorectal distension was measured by counting the number of abdominal contractions recorded by a strain gauge sutured onto the abdominal musculature as described in Gunter et al., Physiol. Behav., 69(3): 379-82 (2000) in awake unrestrained animals. A 5 cm latex balloon catheter inserted via the anal canal into the colon was used for colorectal distensions. Constant pressure tonic distensions were performed in a graded manner (15, 30 or 60 mmHg) and were maintained for a period of 10 min and the numbers of abdominal muscle contractions were recorded to measure the level of colonic sensation. A 10 min recovery was allowed between distensions.

Acetic Acid-Induced Colonic Hypersensitivity

Acetic acid-induced colonic hypersensitivity in rats has been described by Langlois et al., Eur. J. Pharmacol., 318: 141-144 (1996) and Plourde et al., Am. J. Physiol. 273: G191-G196 (1997). In the present study, a low concentration of acetic acid (1.5 ml, 0.6%) was administered intracolonically to sensitize the colon without causing histological damage to the colonic mucosa as described in previous studies (Gunter *et al., supra*).

TESTING

MCI-225 (30 mg/kg; n=6) or vehicle alone (n=4) were administered to the rats intraperitoneally (i.p.) 30 min prior to initiation of the protocol for colorectal distension. Injection volume was 0.2mL using 100% propylene glycol as the vehicle. Three consecutive colorectal distensions at 15, 30 or 60 mmHg applied at 10-min intervals were recorded. Visceromotor responses were evaluated as the number of abdominal muscle contractions recorded during the 10-main periods of colorectal distension. Non-sensitized and sensitized uninjected control animals served to demonstrate the lower and upper levels of response, respectively (n=2/group).

RESULTS

Acetic acid reliably sensitized rat visceromotor responses to CRD (FIG. 1). Vehicle alone had no effect on the response to CRD in acetic acid sensitized animals (FIG. 2). MCI-225 at 30 mg/kg eliminated the visceromotor response to CRD in 50% of the animals (FIG. 1; Responders, n=3).

CONCLUSION

MCI-225 was shown to be effective in a rat model which can be predictive of drug effectiveness in treating IBS in humans. Specifically, as can be seen in FIG. 1, MCI-225 significantly reduced colorectal sensitization-induced increases in visceromotor responses to colorectal distension in 50% of the animals tested. Thus, MCI-225 can be used as a suitable therapy for IBS.

EXAMPLE 2: COMPARISON OF MCI-225, ONDANSETRON AND NISOXETINE IN A MODEL OF VISCEROMOTOR RESPONSE TO COLORECTAL DISTENSION

Additional studies to compare the effects of MCI-225, ondansetron and nisoxetine in the animal model of visceromotor behavioral response to colorectal distension described in Example 1, were conducted.

METHOD

Adult male rats were used in the study. Similar to Example 1, acute colonic hypersensitivity was induced by intracolonic administration of acetic acid and evaluated as an increased number of reflex abdominal muscle contractions induced by colorectal distension. Specifically, rats were anesthetized with Isoflurane (2%) and were instrumented with a strain gauge force transducer for recording of abdominal muscle contractions. A latex balloon and catheter were inserted 11 cm into the colon. The animals were allowed a 30-min period to completely recover from the anesthesia and were then subjected to intracolonic infusion of acetic acid (1.5mL, 0.6%). An additional 30-min period was allowed for sensitization of the colon. At the end of this period, animals received a single dose of either MCI-225 or one of the reference drugs or vehicle via intraperitoneal injection. The protocol for colorectal distension was initiated 30-min post drug administration. After a basal reading of the number of abdominal contractions with the balloon inserted but not distended, three consecutive 10-min lasting colorectal distensions at 15, 30, and 60mmHg were applied at 10-min intervals. Colorectal sensitivity was evaluated by counting the number of reflex abdominal contractions (i.e. the visceromotor response) observed within each distension period.

Animals were randomly assigned to three test groups and dose-dependent controlled experiments were performed as illustrated in Table 1. A control group of animals undergoing the same procedures was treated with vehicle only. Data were summarized for each dose.

Table 1

| Treatment Group | Dose (i.p.) | Number of Subjects |
|---|---|---|
| MCI-225 | 3 mg/kg | 6 |

(continued)

| Treatment Group | Dose (i.p.) | Number of Subjects |
|---|---|---|
| MCI-225 | 10 mg/kg | 6 |
| MCI-225 | 30 mg/kg | 6 |
| Ondansetron | 1 mg/kg | 5 |
| Ondansetron | 5 mg/kg | 5 |
| Ondansetron | 10 mg/kg | 5 |
| Nisoxetine | 3 mg/kg | 6 |
| Nisoxetine | 10 mg/kg | 6 |
| Nisoxetine | 30 mg/kg | 6 |
| Vehicle (100% Propylene Glycol) | 200 $\mu$L | 11 |

MATERIALS

Test and Control Articles

Control drugs for this study were ondansetron and nisoxetine. Ondansetron was supplied from APIN Chemicals LTD. Nisoxetine was supplied by Tocris. MCI-225 was provided by Mitsubishi Pharma Corp. All drugs were dissolved in a vehicle of 100% Propylene Glycol (1,2-Propanediol) by sonicating for a period of 10 minutes. Propylene Glycol was obtained from Sigma Chemical Co.

TESTING

Animals

Adult male Fisher rats were used in this study. The animals were housed two per cage at standard conditions (12hr light/dark cycle, free access to food and water). Following one week of acclimatization to the animal facility, the animals were brought to the laboratory for a second week and handled by the research associate that preformed the experiments. This allowed the animals to become acclimatized to both the experimental environment as well as the research associate who preformed the experiments. All testing procedures used in the study were preapproved.

Acetic Acid-Induced Colonic Hypersensitivity.

Acetic acid-induced colonic hypersensitivity in rats has been described by Langlois *et al.* and Plourde *et al.,* referenced above. In this study low-concentration acetic acid (1.5mL, 0.6%) was administered intracolonically to sensitize the colon without causing histological damage to the colonic mucosa as described in Example 1.

Visceromotor Responses to Colorectal Distension.

The visceromotor behavioral response to colorectal distension was measured by counting the number of abdominal contractions recorded by a strain gauge sutured onto the abdominal musculature as previously described Gunter *et al.,* referenced above. Colorectal distensions were carried out utilizing a 5cm latex balloon catheter inserted into the colon via the anal canal. Constant pressure tonic distensions were performed in a graded manner, i.e., the pressure was increased to the desired level of 15, 30, or 60mmHg and then maintained for a period of 10 minutes during which the number of abdominal contractions were recorded to measure the level of colonic sensation. Ten minute recovery periods were allowed following each distension.

RESULTS AND DISCUSSION

In naive rats, colorectal distensions at graded intraluminal pressure (0, 15, 30 and 60 mmHg) applied for 10 min. with 10 min. intervals between distensions evoked pressure-dependent visceromotor responses. Acetic acid-induced colonic hypersensitivity was characterized by a pressure-dependent linear increase in the number of abdominal contractions compared to non-sensitized controls. In the present study, rats were treated with the test or reference compounds

following colorectal sensitization, thus the obtained drug effects reflect interactions with mechanisms altering the hyper-responsiveness to colonic stimulation without having a preventing effect on the development of colorectal hypersensitivity.

Effect of the reference compounds

Ondansetron, a selective 5-$HT_3$ receptor antagonist, administered at doses of 1,5, or 10 mg/kg, induced a dose-dependent decrease in the number of abdominal contractions. The summarized data presented in FIG. 3 show a significant dose-dependent inhibition of the visceromotor response at all distension pressures compared to the effect of the vehicle. However, even the highest dose of 10 mg/kg ondansetron did not abolish the responses to moderate (30 mmHg) and high (60 mmHg) intraluminal pressure, but rather reduced these responses to levels characteristic for naïve non-sensitized rats. No significant changes in the behavioral activity of the rats were observed following ondansetron treatment.

Nisoxetine, which acts as an inhibitor of noradrenaline re-uptake, had no significant effect on the visceromotor response to colorectal distension when administered at doses of 3, 10 or 30 mg/kg (FIG. 4). However, the high dose of 30 mg/kg nisoxetine was associated with increased exploratory behavior in the home cage during the experiments.

Effect of MCI-225

The summarized effects of the test compound MCI-225 administered at doses of 3,10 or 30 mg/kg and the vehicle are illustrated in FIG. 5. Compared to the vehicle, MCI-225 administered at a dose of 10 mg/kg caused a significant decrease in the number of abdominal contractions recorded in response to colorectal distension at 15, 30 and 60 mmHg. However, the effects of MCI-225 did not show a normal dose-dependent relationship since the high dose of 30 mg/kg MCI-225 appeared to be less effective. In comparison with the reference compounds, the maximal inhibition of visceromotor responses induced by 10 mg/kg MCI-225 was similar to the inhibition caused by 5 mg/kg ondansetron (see FIG. 3).

STATISTICAL ANALYSIS

Statistical significance of the treatment groups was assessed using one-way ANOVA followed by Tukey post-test. Differences between responses observed in vehicle treated and drug treated rats were considered significant at $p<0.05$. (*) $p<0.05$, (**) $p<0.01$, (***) $p<0.001$

CONCLUSION

MCI-225, was shown to be effective in a rat model which can be predictive of drug effectiveness in treating IBS in humans. Specifically, as can be seen FIG. 5, MCI-225 significantly reduced the number of abdominal contractions recorded in response to colorectal distension at various pressures.

EXAMPLE 3: EFFECT OF MCI-225 IN A MODEL OF INCREASED COLONIC TRANSIT

METHOD

The model used in this example provided a method of determining the ability of MCI-225 to normalize accelerated colonic transit induced by water avoidance stress (WAS). Ondansetron (5-$HT_3$ receptor antagonist), nisoxetine (NARI) and a combination of ondansetron and nisoxetine were used as comparison compounds. The model provides a method of evaluating the effectiveness of a compound in a specific patient group of IBS sufferers where stress induced colonic motility is considered a significant contributing factor.

Preliminary testing in the water avoidance stress model confirmed that there exists an association between stress and altered colonic motility. Fecal pellet output was measured by counting the total number of fecal pellets produced during 1 hour of WAS. Using the WAS model, the effect of MCI-225 was compared to the effects of ondansetron (5-$HT_3$ antagonist) or nisoxetine (noradrenaline reuptake inhibitor -NARI) to affect fecal pellet output. The results showed that MCI-225 inhibited stress-induced accelerated colonic transit and can therefore be effective in the treatment of IBS, particularly IBS where stress induced colonic motility is considered a significant contributing factor.

TESTING

Animals

Adult male F-344 rats, supplied by Charles River Laboratories and weighing 270-350 g, were used to complete

this study. The rats were housed 2 per cage under standard conditions. Following one to two weeks of acclimatization to the animal facility, the rats were brought to the laboratory and handled daily for another week to acclimatize them to laboratory conditions and to the research associate who performed the studies. All procedures used in this study were approved in accordance with facility standards.

Acclimatization prior to experiments

All rats underwent sham stress (1-hour in stress chamber without water) for 2-4 consecutive days before undergoing WAS (sham was performed until rats produced 0-1 pellet per hour for 2 consecutive days). At the end of the 1-hour stress period, the fecal pellets were counted and recorded.

Procedure

WAS causes an acceleration of colonic transit, which can be quantified by counting the number of fecal pellets, produced during the stress procedure. Rats were placed for 1-hour into a stress chamber onto a raised platform 7.5 cm x 7.5 cm x 9cm (L x W x H) in the center of a stress chamber filled with room temperature water 8 cm in depth. The stress chamber was constructed from a rectangular plastic tub (40.2 x 60.2 x 31.2 cm). The a summary of the treatment and control groups is set forth in Table 2.

Table 2

| Treatment Group | Dose (i.p.) | Number of subjects |
|---|---|---|
| MCI-225 | 3 mg/kg | 8 |
| MCI-225, | 10 mg/kg | 8 |
| MCI-225 | 30 mg/kg | 8 |
| Ondansetron | 1 mg/kg | 8 |
| Ondansetron | 5 mg/kg | 8 |
| Ondansetron | 10 mg/kg | 8 |
| Nisoxetine | 3 mg/kg | 8 |
| Nisoxetine | 10 mg/kg | 8 |
| Nisoxetine | 30 mg/kg | 8 |
| Control Group Home Cage | n/a | 8 |
| Control Group Sham Stress | n/a | 8 |
| Control Group WAS | n/a | 8 |
| Control Group Vehicle (100% Propylene Glycol) | 200 μL | 8 |

MATERIALS

Test and Control Articles

Control drugs for this study were ondansetron and nisoxetine. Ondansetron was supplied from APIN Chemicals LTD. Nisoxetine was supplied by Tocris. MCI-225 was provided by Mitsubishi Pharma Corp. All drugs were dissolved in a vehicle of 100% propylene glycol (1,2-propanediol) by sonicating for a period of 10 minutes. Propylene glycol was obtained from Sigma Chemical Co. MCI-225 and nisoxetine were tested at doses of 3, 10 and 30 mg/kg and ondansetron was tested at doses of 1, 5 and 10 mg/kg. All drugs and the vehicle were administered as an i.p. injection in a volume of 0.2 mL.

RESULTS AND DISCUSSION

Controls

As illustrated in FIG. 6, there was no significant difference in the number of fecal pellets produced in 1 hour

between the animals in their home cage or the sham stress control group. As expected, upon exposure to a WAS (WAS basal) for 1 hour, there was a highly significant ($p<0.001$) increase in fecal pellet output compared to fecal pellet output from rats in their home cage or the sham stress control group. After acclimation to the stress chamber for 2-4 days the fecal pellet output of the WAS vehicle treatment group was not statistically different from the fecal pellet output of the non-treated WAS group.

Treatment Groups- MCI-225

In rats pretreated with MCI-225 (dosed at 3, 10 or 30 mg/kg i.p.) and then placed on the WAS, the number of fecal pellets produced during 1 hour was significantly less than the number produced during WAS in the vehicle treated group. As illustrated in FIG. 7, MCI-225 caused a significant dose-dependent inhibition of WAS-induced fecal pellet output at all doses.

Nisoxetine

As illustrated in FIG. 8, the number of fecal pellets produced during 1 hour of WAS was reduced by all doses (3, 10 and 30 mg/kg i.p.) of Nisoxetine. However, when compared to the vehicle treated group there was significantly fewer fecal pellet produced during WAS at doses of 10 and 30 mg/kg of Nisoxetine.

Ondansetron

Ondansetron caused a dose-dependent inhibition of the stress-induced fecal pellet output. As illustrated in FIG. 9, the number of fecal pellets produced during 1 hour of WAS in all ondansetron treatment groups (1, 5 and 10 mg/kg i.p.) was significantly less than the number produced during WAS in the vehicle treatment group.

Combination of Nisoxetine and Ondansetron

For the combination treatment group, the doses of nisoxetine and ondansetron that displayed the most efficacy when dosed alone were used. When nisoxetine (30 mg/kg) was dosed in combination with ondansetron (10 mg/kg), the number of fecal pellets produced during 1 hour of WAS was significantly less than the number produced during WAS in the vehicle control group ($p<0.01$). The results are set forth graphically in FIG. 10.

STATISTICAL ANALYSIS

Statistical significance was assessed using one-way ANOVA followed by Tukey post-test. Statistical differences were compared between the WAS groups and the sham stress group and was considered significant if $p<0.05$. (*) $p<0.05$, (**) $p<0.01$, (***) $P<0.001$

CONCLUSION

These experiments demonstrated that stress, in this case a water avoidance stressor, caused a significant increase in colonic transit as demonstrated by an increase in fecal pellet output. The overall conclusion is that MCI-225 significantly inhibited the stress-induced increase in fecal pellet production to an extent that resembled that observed with either nisoxetine or ondansetron. Thus, MCI-225 can be used as a suitable therapy for the treatment of non-constipated IBS.

EXAMPLE 4: EFFECT OF MCI-225 ON SMALL INTESTINAL TRANSIT

The effect of MCI-225 on the inhibition of small intestinal transit was evaluated and compared to results obtained using ondansetron, nisoxetine and a combination of ondansetron and nisoxetine using the Small Intestinal Transit Rodent Model described below.

METHOD

Specifically, the effects of MCI-225, the reference compounds (ondansetron and nisoxetine) and the vehicle on small intestinal transit were investigated in rats under control conditions. Following an overnight fast, rats were brought to the laboratory in their home cages and received an i.p. injection of one of the following: MCI-225, 100% propylene glycol (vehicle), ondansetron, nisoxetine and a combination of ondansetron and nisoxetine. Control rats received no

treatment. The treated rats were placed back in the home cages and after 30 min, were fed a 2 mL charcoal meal via an oral gavage. Small intestinal transit was measured following 15 min test-period. Each rat was placed briefly in a glass chamber with IsoFlo for anesthesia and sacrificed. The stomach and the small intestine were removed and the total length of the small intestine was measured. Transit was then measured as the distance that the charcoal meal had traveled along the small intestine and expressed as % of the total length. Animals were randomly assigned to experimental groups and experiments were performed as illustrated in Table 3.

Table 3

| Treatment Group | Dose (i.p.) | Number of subjects | Mean | Standard Deviation | Standard Error to the Mean (SEM) |
|---|---|---|---|---|---|
| MCI-225 | 3 mg/kg | 6 | 30.1% | 20.3% | 8.3% |
| MCI-225 | 10 mg/kg | 5 | 4.2% | 5.8% | 2.6% |
| MCI-225 | 30 mg/kg | 4 | 8.8% | 7.1% | 3.5% |
| Ondansetron | 1 mg/kg | 5 | 27.6% | 16.0% | 7.2% |
| Ondansetron | 5 mg/kg | 5 | 32.1% | 15.6% | 7.0% |
| Ondansetron | 10 mg/kg | 5 | 18.4%% | 11.8% | 5.3% |
| Nisoxetine | 3 mg/kg | 6 | 40.3% | 12.2% | 5.0% |
| Nisoxetine | 10 mg/kg | 5 | 38.6% | 26.7% | 11.9% |
| Nisoxetine | 30 mg/kg | 5 | 4.7% | 1.05% | 4.7% |
| Nisoxetine and Ondansetron | 10 mg/kg 5 mg/kg | 5 | 14.2% | 11.8% | 5.3% |
| Control Group Vehicle (100% Propylene Glycol) | 200 μL | 5 | 56.0% | 8.0% | 3.6% |
| Naive rats (untreated) | n/a | 5 | 74.6% | 12.4% | 5.6% |

MATERIALS

Test and Control Articles

Ondansetron was supplied from APIN Chemicals LTD. Nisoxetine was supplied by Tocris. MCI-225 was provided by Mitsubishi Pharma Corp. All drugs were dissolved in a vehicle of 100% propylene glycol (1,2-Propanediol) by sonicating for 10 min. Propylene glycol was obtained from Sigma Chemical Co. Ondansetron, a 5-$HT_3$-receptor antagonist was dosed i.p. at 1, 5, and 10mg/kg. Nisoxetine was administered i.p. at 3,10, and 30mg/kg. All doses were delivered in a final volume of 200 μL. Animals in the vehicle control group received 200 μL of 100% Propylene glycol and animals in the normal control group were untreated.

TESTING

Animals

Adult male F-344 rats (230-330g) were used in the study. The rats were housed 2 per cage under standard conditions. The animals were fed a standard rodent diet and food and water were provided "ad libitum". Rats were allowed to acclimatize to the animal facility for one week prior to the transit experiments. All procedures used in this study were pre-approved.

Small intestinal transit in rats was investigated by the passage of a charcoal meal along the small intestine during a defined time period (15-min). The animals were deprived of food for 12-16 hrs prior to the experiments. Rats were given a charcoal meal (a mixture of charcoal, gum arabic, and distilled water) as a 2 mL oral gavage and were sacrificed after a 15-min test period. The distance traveled by the charcoal meal was quantified as a percent of the small

intestinal length, using the following equation:

$$\text{Transit (\%)} = \text{cm traveled by meal/ cm total small intestinal length} \times 100$$

DATA AND STATISTICAL ANALYSIS

Small intestinal transit was evaluated in relative units (%) of the total intestinal length in the following groups receiving different drug treatment: naïve (untreated), vehicle (propylene glycol, 200 μL i.p.), MCI-225 (3, 10 and 30 mg/kg, i.p.), nisoxetine (3, 10 and 30 mg/kg, i.p.), ondansetron (1, 5 and 10 mg/kg, i.p.) and a combination of nisoxetine (10 mg/kg, i.p.) and ondansetron (5 mg/kg, i.p.). A total of 61 experiments were performed (4-6 rats per group).

Statistical analysis was performed to determine mean, standard error to the mean and standard deviation for each group (See Table 3). FIGS. 11-15 are based on the data. Differences between individual dose groups within treatments and comparisons between drug-treated and vehicle-treated groups were determined using an unpaired *t* test considering that when % is used as a relative unit, *t*-statistic is relevant. In all cases $p < 0.05$ was considered statistically significant. Statistical significance is indicated as $p<0.05$. (*) $p<0.05$, (**) $p<0.01$, (***) $p<0.001$ in FIGS. 11-15.

RESULTS AND DISCUSSION

In naïve untreated rats the charcoal meal reached a distance of 75 ± 12 % of the total length during the 15-min test period. In comparison, when rats received an i.p. injection of the vehicle 30-min prior to receiving the charcoal meal, the small intestinal transit measured under the same conditions was reduced to 56 ± 8% of the total length of the small intestine. Data from the study are summarized in FIG. 11. However, the vehicle-treated animals showed uniform and reproducible values of small intestinal transport, which served as control to evaluate the effect of drug treatment.

Effect of MCI-225

A series of experiments was performed to establish the effect of increasing doses of 3, 10 or 30 mg/kg MCI-225 on small intestinal transport. Data from the study are summarized in FIG. 12. Compared to the vehicle, MCI-225 induced a dose-dependent inhibition of small intestinal transit with a maximal reduction of the distance traveled by the charcoal meal to 4.2 ± 2.6% of the total length of the small intestine at a dose of 10 mg/kg.

Effects of the reference compounds

In separate studies animals were treated with increasing doses of 1, 10 or 30 mg/kg nisoxetine, which blocks noradrenaline re-uptake. When administered at doses of 3 or 10 mg/kg nisoxetine showed a tendency to decrease small intestinal transit, while a dose of 30 mg/kg almost completely inhibited the transit (FIG. 13). The effect of ondansetron administered at doses of 1, 5 or 10 mg/kg was also investigated. As illustrated in FIG. 14, ondansetron caused a significant reduction in small intestinal transit, without showing a normal dose-dependent relationship.

The inhibition caused by nisoxetine was considered the result of delayed stomach emptying, since the charcoal meal was completely retained in the stomach in 4 out of 5 animals following a dose of 30 mg/kg nisoxetine. This effect differed from the effects found with ondansetron or MCI-225, where a portion of the charcoal meal was always found to advance from the stomach into the small intestine. When 5 mg/kg ondansetron and 10 mg/kg nisoxetine were injected simultaneously the drugs showed a reduction in the distance traveled by the meal of 14% of the total length of the small intestine (FIG. 15) (i.e. the maximal effect of the combination was greater (lower % values) compared to the effects of the individual doses of 5 mg/kg ondansetron or 10 mg/kg nisoxetine). These findings establish that the decrease in small intestinal transit induced by MCI-225 can result from combined effects on 5-$HT_3$ receptors and noradrenaline re-uptake mechanisms.

While this invention has been particularly shown and described with references to preferred embodiments thereof, it will be understood by those skilled in the art that various changes in form and details may be made therein without departing from the scope of the invention encompassed by the appended claims.

Certain embodiments of the invention are now described in the paragraphs below.

1. A method of treating a functional bowel disorder in a subject in need thereof comprising administering to said subject a therapeutically effective amount of a compound of Formula I:

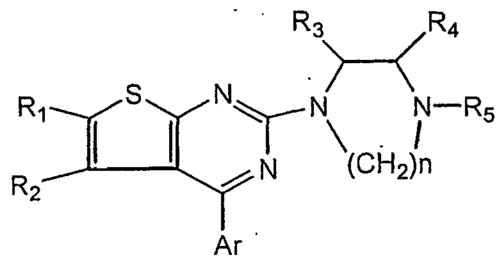

I

wherein, $R_1$ and $R_2$ independently represent hydrogen, halogen or a $C_1$-$C_6$ alkyl group; or $R_1$, and $R_2$ together with the carbon atom to which they are attached form a cycloalkylene group having 5 to 6 carbon atoms;
$R_3$ and $R_4$ independently represent hydrogen or a $C_1$-$C_6$ alkyl group;
$R_5$ is hydrogen, $C_1$-$C_6$ alkyl,

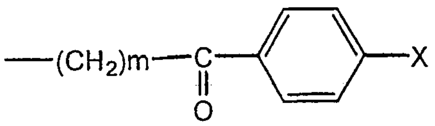

,

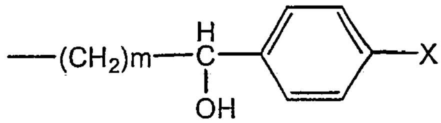

or -C(O)-NH-$R_6$
wherein m is an integer from about 1 to about 3, X is halogen and $R_6$ is a $C_1$-$C_6$ alkyl group; and

Ar is a substituted or unsubstituted phenyl, 2-thienyl or 3-thienyl group; and
n is 2 or 3; or a pharmaceutically acceptable salt thereof.

Paragraph 2. The method of Paragraph 1, wherein the functional bowel disorder is irritable bowel syndrome.

Paragraph 3. The method of Paragraph 2, wherein the irritable bowel syndrome is diarrhea predominant irritable bowel syndrome.

Paragraph 4. The method of Paragraph irritable bowel syndrome is alternating constipation/diarrhea irritable bowel syndrome.

Paragraph 5. The method of Paragraph 2, wherein the irritable bowel syndrome is nonconstipated irritable bowel syndrome.

Paragraph 6. The method of Paragraph 1, wherein the subject is a human.

Paragraph 7. The method of Paragraph 1, wherein for the compound of formula I, $R_1$ is a $C_1$-$C_6$ alkyl group and Ar is a substituted phenyl.

Paragraph 8. The method of Paragraph 7, wherein the substituted phenyl group is substituted with a halogen.

Paragraph 9. The method of Paragraph 1, wherein for the compound of Formula I n is 2, $R_1$ is a $C_1$-$C_6$ alkyl group and Ar is a substituted phenyl.

Paragraph 10. The method of Paragraph 9, wherein the substituted phenyl group is substituted with a halogen and $R_1$ is a methyl group.

Paragraph 11. The method of Paragraph 1, wherein for the compound of Formula I, $R_1$ is a $C_1$-$C_6$ alkyl group or a halogen and Ar is an unsubstituted phenyl.

Paragraph 12. The method of Paragraph 11, wherein $R_2$ is hydrogen or a $C_1$-$C_6$ alkyl group.

Paragraph 13. The method of Paragraph 1, wherein for the compound of Formula I, n is 2, $R_1$, is a $C_1$-$C_6$ alkyl group and Ar is an unsubstituted phenyl.

Paragraph 14. The method of Paragraph 13, wherein $R_2$ is hydrogen or a $C_1$-$C_6$ alkyl group

Paragraph 15. A method of treating a functional bowel disorder in a subject in need thereof comprising administering to said subject a therapeutically effective amount of
a compound represented by Formula II:

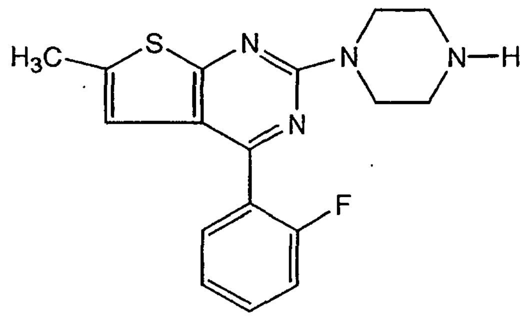

II

or a pharmaceutically acceptable salt thereof.

Paragraph 16. The method of Paragraph 15, wherein the functional bowel disorder is irritable bowel syndrome.

Paragraph 17. The method of Paragraph16, wherein the irritable bowel syndrome is diarrhea predominant irritable bowel syndrome, alternating constipation/diarrhea irritable bowel syndrome or nonconstipated irritable bowel syndrome.

Paragraph 18. The method of Paragraph 15, wherein the subject is a human.

Paragraph 19. A method of treating diarrhea predominant irritable bowel syndrome in a subject in need thereof comprising administering to said subject a therapeutically effective amount of a compound represented by Formula II:

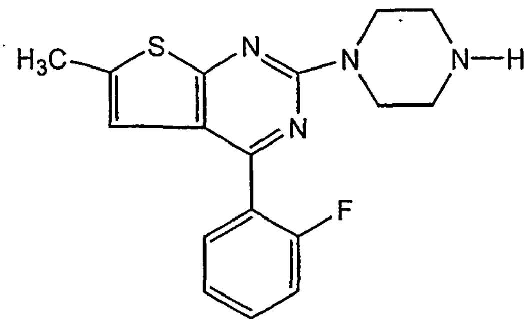

II

or a pharmaceutically acceptable salt thereof.

paragraphe 20. The method of Paragraph 19, wherein the subject is a human.

Paragraph 21. A method for treating a functional bowel disorder in a subject in need thereof comprising administering to said subject:

a) a first amount of a $5\text{-}HT_3$ receptor antagonist; and

b) a second amount of a noradrenaline reuptake inhibitor

wherein the first and second amounts together comprise a therapeutically effective amount.

Paragraph 22. The method of Pargraph 21, wherein the functional bowel disorder is irritable bowel syndrome.

Paragraphe 23. The method of Paragraph 22, wherein the irritable bowel syndrome is diarrhea predominant irritable bowel syndrome.

Paragraphe 24. The method of Paragraph 12, wherein the irritable bowel syndrome is alternating constipation/ diarrhea irritable bowel syndrome.

Paragraphe 25. The method of Paragraph 22, wherein the irritable bowel syndrome is nonconstipated irritable bowel syndrome.

Paragraphe 26. The method of Paragraph 21, wherein the subject is a human.

Paragraphe 27. The method of Paragraph 21, wherein the 5-$HT_3$ receptor antagonist is selected from the group consisting of indisetron, YM-114 ((R)-2,3-dihydro-1-[(4,5,6,7-tetrahydro-1H-benzimidazol-5-yl-)carbonyl]-1H-indole), granisetron, talipexole, azasetron, bemesetron, tropisetron, ramosetron, ondansetron, palonosetron, lerisetron, alosetron, N-3389, zacopride, cilansetron, E-3620 ([3(S)-endo]-4-amino-5-chloro-N-(8-methyl-8-azabicyclo [3.2.1-]oct-3-yl-2[(1-methyl-2-butynyl)oxy]benzamide), lintopride, KAE-393, itasetron, zatosetron, dolasetron, (±)-zacopride, (±)-renzapride, (-)-YM-060, DAU-6236, BIMU-8 and GK-128 [2-[2-methylimidazol-1-yl)methyl]-benzo[*f*]thiochromen-1-one monohydrochloride hemihydrate].

Paragraphe 28. The method of Paragraph 27, wherein in the 5-$HT_3$ receptor antagonist is selected from the group consisiting of indisetron, granisetron, azasetron, bemesetron, tropisetron, ramosetron, ondansetron, palonosetron, lerisetron, alosetron, cilansetron, itasetron, zatosetron, and dolasetron.

Paragraphe 29. The method of Paragraph 21, wherein the noradrenaline reuptake inhibitor is selected from the group consisting of venlafaxine, duloxetine, buproprion, milnacipran, reboxetine, lefepramine, desipramine, nortriptyline, tomoxetine, maprotiline, oxaprotiline, levoprotiline, viloxazine and atomoxetine.

Paragraphe 30. The method of Paragraph 29, wherein the noradrenaline reuptake inhibitor is selected from the group consisting of reboxetine, lefepramine, desipramine, nortriptyline, tomoxetine, maprotiline, oxaprotiline, levoprotiline, viloxazine and atomoxetine.

Paragraph 31. A method for treating a functional bowel disorder in a subject in need thereof comprising administering to said subject:

a) a therapeutically effective amount of a 5-$HT_3$ receptor antagonist; and

b) a therapeutically effective amount of a noradrenaline reuptake inhibitor.

Paragraphe 32. The method of Paragraph 31, wherein the functional bowel disorder is irritable bowel syndrome.

Paragraphe 33. The method of Paragraph 32, wherein the irritable bowel syndrome is diarrhea predominant irritable bowel syndrome.

Paragraphe 34. The method of Paragraph 32, wherein the irritable bowel syndrome is alternating constipation/ diarrhea irritable bowel syndrome.

Paragraphe 35. The method of Paragraph 32, wherein the irritable bowel syndrome is nonconstipated irritable bowel syndrome.

Paragraphe 36. The method of Paragraph 31, wherein the subject is a human.

Paragraphe 37. The method of Paragraph 31 5-$HT_3$ receptor antagonist is selected from the group consisting of

indisetron, YM-114 ((R)-2,3-dihydro-1-[(4,5,6,7-tetrahydro-1H-benzimidazol-5-yl-)carbonyl]-1H-indole), granisetron, talipexole, azasetron, bemesetron, tropisetron, ramosetron, ondansetron, palonosetron, lerisetron, alosetron, N-3389, zacopride, cilansetron, E-3620 ([3(S)-endo]-4-amino-5-chloro-N-(8-methyl-8-azabicyclo[3.2.1-] oct-3-yl-2[(1-methyl-2-butynyl)oxy]benzamide), lintopride, KAE-393, itasetron, zatosetron, dolasetron, (±)-zacopride, (±)-renzapride, (-)-YM-060, DAU-6236, BIMU-8 and GK-128 [2-[2-methylimidazol-1-yl)methyl]-benzo[*f*]thiochromen-1-one monohydrochloride hemihydrate].

Paragraphe 38. The method of Paragraph 37, wherein the 5-$HT_3$ receptor antagonist is selected from the group consisiting of indisetron, granisetron, azasetron, bemesetron, tropisetron, ramosetron, ondansetron, palonosetron, lerisetron, alosetron, cilansetron, itasetron, zatosetron, and dolasetron.

Paragraph 39. The method of Paragraph 31, wherein the noradrenaline reuptake inhibitor is selected from the group consisting of venlafaxine, duloxetine, buproprion, milnacipran, reboxetine, lefepramine, desipramine, nortriptyline, tomoxetine, maprotiline, oxaprotiline, levoprotiline, viloxazine and atomoxetine.

Paragraph 40. The method of Paragraph 39, wherein the noradrenaline reuptake inhibitor is selected from the group consisting of reboxetine, lefepramine, desipramine, nortriptyline, tomoxetine, maprotiline, oxaprotiline, levoprotiline, viloxazine and atomoxetine.

Paragraph 41. A method of treating a functional bowel disorder in a subject in need thereof comprising administering to said subject a therapeutically effective amount of
a noradrenaline reuptake inhibitor, wherein the noradrenaline reuptake inhibitor characterized by the substantial absence of anticholinergic effects.

Paragraph 42. The method of Paragraph 41, wherein the functional bowel disorder is irritable bowel syndrome.

Paragraph 43. The method of Paragraph 42, wherein the irritable bowel syndrome is diarrhea predominant irritable bowel syndrome.

Paragraph 44. The method of Paragraph 42, wherein the irritable bowel syndrome is alternating constipation/diarrhea irritable bowel syndrome.

Paragraph 45. The method of Paragraph 42, wherein the irritable bowel syndrome is nonconstipated irritable bowel syndrome.

Paragraph 46. The method of Paragraph 41, wherein the subject is a human.

Paragraph 47. The method of Paragraph 41, wherein the noradrenaline reuptake inhibitor is selected from the group consisting of venlafaxine, duloxetine, buproprion, milnacipran, reboxetine, lefepramine, desipramine, nortriptyline, tomoxetine, maprotiline, oxaprotiline, levoprotiline, viloxazine and atomoxetine.

Paragraph 48. The method of Paragraph 47, wherein the noradrenaline reuptake inhibitor is selected from the group consisting of reboxetine, lefepramine, desipramine, nortriptyline, tomoxetine, maprotiline, oxaprotiline, levoprotiline, viloxazine and atomoxetine.

Paragraph 49. A pharmaceutical composition comprising:

a) a first amount of a 5-$HT_3$ receptor antagonist; and
b) a second amount of a noradrenaline reuptake inhibitor.

Paragraph 50. The pharmaceutical composition of Paragraph 49, further comprising a pharmaceutically acceptable carrier.

Paragraph 51. The pharmaceutical composition of Paragraph 49, wherein the 5-$HT_3$ receptor antagonist is selected from the group consisting of indisetron, YM-114 ((R)-2,3-dihydro-1-[(4,5,6,7-tetrahydro-1H-benzimidazol-5-yl-)carbonyl]-1H-indole), granisetron, talipexole, azasetron, bemesetron, tropisetron, ramosetron, ondansetron, palonosetron, lerisetron, alosetron, N-3389, zacopride, cilansetron, E-3620 ([3(S)-endo]-4-amino-5-chloro-N-(8-methyl-8-azabicyclo [3.2.1-] oct-3-yl-2[(1-methyl-2-butynyl)oxy]benzamide), lintopride, KAE-393, itasetron, zatosetron, dolasetron, (±)-zacopride, (±)-renzapride, (-)-YM-060, DAU-6236, BIMU-8 and GK-128 [2-[2-methylimidazol-1-yl)methyl]-benzo[*f*]thiochromen-1-one monohydrochloride hemihydrate].

Paragraph 52. The pharmaceutical composition of Paragraph 51, wherein the 5-$HT_3$ receptor antagonist is selected from the group consisting of indisetron, granisetron, azasetron, bemesetron, tropisetron, ramosetron, ondansetron, palonosetron, lerisetron, alosetron, cilansetron, itasetron, zatosetron, and dolasetron.

Paragraph 53. The pharmaceutical composition of Paragraph 49, wherein the noradrenaline reuptake inhibitor is selected from the group consisting ofvenlafaxine, duloxetine, buproprion, milnacipran, reboxetine, lefepramine, desipramine, nortriptyline, tomoxetine, maprotiline, oxaprotiline, levoprotiline, viloxazine and atomoxetine.

Paragraph 54. The pharmaceutical composition of Paragraph 53, wherein the noradrenaline reuptake inhibitor is selected from the group consisting of reboxetine, lefepramine, desipramine, nortriptyline, tomoxetine, maprotiline, oxaprotiline, levoprotiline, viloxazine and atomoxetine.

Paragraph 55. A method for processing a claim under a health insurance policy submitted by a claimant seeking reimbursement for costs associated with treatment of a functional bowel disorder, wherein said treatment comprises coadministering to a subject a first amount of a 5-$HT_3$ receptor antagonist and a second amount of a noradrenaline reuptake inhibitor, wherein the first and second amounts together comprise a therapeutically effective amount comprising:

a) reviewing said claim;
b) determining whether said treatment is reimbursable under said insurance policy; and
c) processing said claim to provide partial or complete reimbursement of said costs.

Paragraph 56. The method of Paragraph 55, wherein the functional bowel disorder is irritable bowel syndrome.

Paragraph 57. The method of Paragraph 56, wherein the irritable bowel syndrome is diarrhea predominant irritable bowel syndrome.

Paragraph 58. The method of Paragraph 56, wherein the irritable bowel syndrome is alternating constipation/diarrhea irritable bowel syndrome.

Paragraph 59. A method for processing a claim under a health insurance policy submitted by a claimant seeking reimbursement for costs associated with treatment of a functional bowel disorder, wherein said treatment comprises coadministering to a subject a therapeutically effective amount of a 5-$HT_3$ receptor antagonist and a therapeutically effective amount of a noradrenaline reuptake inhibitor comprising:

a) reviewing said claim;
b) determining whether said treatment is reimbursable under said insurance policy; and
c) processing said claim to provide partial or complete reimbursement of said costs.

Paragraph 60. The method of Paragraph 59, wherein the functional bowel disorder is irritable bowel syndrome.

Paragraph 61. The method Paragraph 60, irritable bowel syndrome is diarrhea predominant irritable bowel syndrome.

Paragraph 62. The method of Paragraph 60, wherein the irritable bowel syndrome is alternating constipation/diarrhea irritable bowel syndrome.

## Claims

**1.** A compound of Formula I or Formula II:

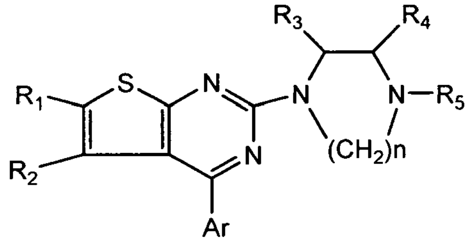

I

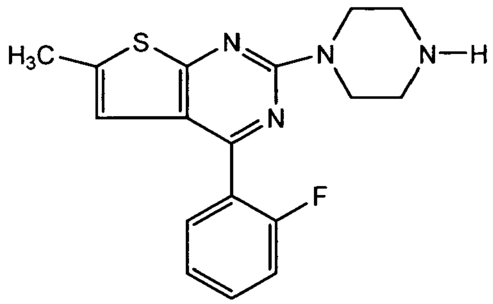

II

wherein, $R_1$ and $R_2$ independently represent hydrogen, halogen or a $C_1$-$C_6$ alkyl group; or $R_1$ and $R_2$ together with the carbon atom to which they are attached form a cycloalkylene group having 5 to 6 carbon atoms;
$R_3$ and $R_4$ independently represent hydrogen or a $C_1$-$C_6$ alkyl group;
$R_5$ is hydrogen, $C_1$-$C_6$ alkyl,

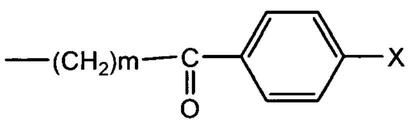

,

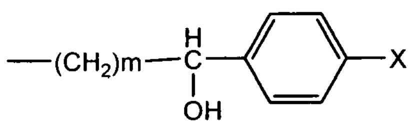

or -C(O)-NH-$R_6$
wherein m is an integer from about 1 to about 3, X is halogen and $R_6$ is a $C_1$-$C_6$ alkyl group; and
Ar is a substituted or unsubstituted phenyl, 2-thienyl or 3-thienyl group; and
n is 2 or 3; or a pharmaceutically acceptable salt thereof,
for use in the treatment of a functional bowel disorder.

**2.** Use of a compound selected from the group consisting of:

indisetron; YM-114 ((R)-2,3-dihydro-1-[(4,5,6,7-tetrahydro-1H-benzimidazol-5-yl-)carbonyl]-1H-indole); granisetron; talipexole; azasetron; bemesetron; tropisetron; ramosetron; ondansetron; palonosetron; lerisetron; alosetron; N-3389; zacopride; cilansetron; E-3620 ([3(S)-endo]-4-amino-5-chloro-N-(8-methyl-8-azabicyclo[3.2.1-]oct-3-yl-2[(1-methyl-2-butynyl)oxy]benzamide); lintopride; KAE-393; itasetron; zatosetron; dolasetron; (±)-zacopride; (±)-renzapride; (-)-YM-060; DAU-6236; BIMU-8; and GK-128 [2-[2-methylimidazol-1-yl)methyl]-benzo[*f*]thiochromen-1-one monohydrochloride hemihydrate],
for the manufacture of a medicament for the treatment of a functional bowel disorder in a subject who is also administered a compound selected from the group consisting of:
venlafaxine; duloxetine; buproprion; milnacipran; reboxetine; lefepramine; desipramine; nortriptyline; tomoxetine; maprotiline; oxaprotiline; levoprotiline; viloxazine; and atomoxetine.

**3.** Use of a compound selected from the group consisting of venlafaxine, duloxetine, buproprion, milnacipran, reboxetine, lefepramine, desipramine, nortriptyline, tomoxetine, maprotiline, oxaprotiline, levoprotiline, viloxazine, and atomoxetine for the manufacture of a medicament for the treatment of a functional bowel disorder.

**4.** A pharmaceutical composition comprising:

a) a compound selected from the group consisting of indisetron, YM-114 ((R)-2,3-dihydro-1-[(4,5,6,7-tetrahydro-1H-benzimidazol-5-yl-)carbonyl]-1H-indole), granisetron, talipexole, azasetron, bemesetron, tropisetron, ramosetron, ondansetron, palonosetron, lerisetron, alosetron, N-3389, zacopride, cilansetron, E-3620 ([3(S)-endo]-4-amino-5-chloro-N-(8-methyl-8-azabicyclo[3.2.1-]oct-3-yl-2[(1-methyl-2-butynyl)oxy]benzamide), lintopride, KAE-393, itasetron, zatosetron, dolasetron, (±)-zacopride, (±)-renzapride, (-)-YM-060, DAU-6236, BIMU-8, and GK-128 [2-[2-methylimidazol-1-yl)methyl]-benzo[*f*]thiochromen-1-one monohydrochloride hemihydrate];
b) a compound selected from the group consisting of venlafaxine, duloxetine, buproprion, milnacipran, reboxetine, lefepramine, desipramine, nortriptyline, tomoxetine, maprotiline, oxaprotiline, levoprotiline, viloxazine, and atomoxetine; and, optionally
c) a pharmaceutically acceptable carrier.

**5.** A kit comprising:

a) a first compound selected from the group consisting of indisetron, YM-114 ((R)-2,3-dihydro-1-[(4,5,6,7-tetrahydro-1H-benzimidazol-5-yl-)carbonyl]-1H-indole), granisetron, talipexole, azasetron, bemesetron, tropisetron, ramosetron, ondansetron, palonosetron, lerisetron, alosetron, N-3389, zacopride, cilansetron, E-3620 ([3(S)-endo]-4-amino-5-chloro-N-(8-methyl-8-azabicyclo[3.2.1-]oct-3-yl-2[(1-methyl-2-butynyl)oxy]benzamide), lintopride, KAE-393, itasetron, zatosetron, dolasetron, (±)-zacopride, (±)-renzapride, (-)-YM-060, DAU-6236, BIMU-8, and GK-128 [2-[2-methylimidazol-1-yl)methyl]-benzo[*f*]thiochromen-1-one monohydrochloride hemihydrate];
b) a second compound selected from the group consisting of venlafaxine, duloxetine, buproprion, milnacipran, reboxetine, lefepramine, desipramine, nortriptyline, tomoxetine, maprotiline, oxaprotiline, levoprotiline, viloxazine, and atomoxetine; and
c) an instruction insert for coadministering the compounds.

**6.** Compound for use according to Claim 1, or use according to Claim 2 or Claim 3, wherein the functional bowel disorder is irritable bowel syndrome, such as:

(i) diarrhea predominant irritable bowel syndrome;
(ii) alternating constipation/diarrhea irritable bowel syndrome; or
(iii) nonconstipated irritable bowel syndrome.

**7.** Compound for use, or use, according to Claim 6, wherein the irritable bowel syndrome is diarrhea predominant irritable bowel syndrome.

**8.** Compound for use according to Claim 1, wherein for the compound of Formula I:

(i) n is 2, $R_1$ is a methyl group and Ar is a phenyl that is substituted with a halogen; or
(ii) n is 2, $R_1$ is a $C_1$-$C_6$ alkyl group, $R_2$ is hydrogen or a $C_1$-$C_6$ alkyl group and Ar is an unsubstituted phenyl.

**9.** Compound for use according to Claim 1, wherein the compound is for use in the treatment of a functional bowel disorder or diarrhea predominant irritable bowel syndrome by administration of

about 0.001 mg to about 1000 mg,
about 0.05 mg to about 500 mg,
about 0.03 mg to about 300 mg,
about 0.02 mg to about 200 mg,
about 0.1 mg to about 50 mg, or
about 0.5 mg to about 10 mg

per day of the compound or a pharmaceutically acceptable salt thereof.

**10.** Compound for use according to Claim 9, wherein the compound for use in the treatment of or diarrhea predominant irritable bowel syndrome by administration of 0.5 mg to 10 mg per day of the compound or a pharmaceutically acceptable salt thereof.

**11.** Compound for use according to Claim 9 or Claim 10, wherein the compound is of Formula II.

**12.** Compound for use according to any of Claims 9 to 11, wherein the dose per day is administered in a single dosage.

**13.** Compound for use according to any of Claims 9 to 11, wherein the dose per day is administered in multiple dosages.

**14.** Compound for use according to Claim 1, wherein the pharmaceutically acceptable salt of the compound of Formula I or Formula II is a salt, or a hydrate thereof, with an acid selected from hydrochloric, hydrobromic, hydroiodic, nitric, sulfuric, phosphoric, formic, acetic, propionic, succinic, camphorsulfonic, citric, fumaric, gluconic, isethionic, lactic, malic, mucic, tartaric, para-toluenesulfonic, glycolic, glucuronic, maleic, furoic, glutamic, benzoic, anthranilic, salicylic, phenylacetic, mandelic, embonic, methanesulfonic, ethanesulfonic, pantothenic, benzenesulfonic, stearic, sulfanilic, alginic and galacturonic acid.

**15.** Use of a compound of Formula I or Formula II:

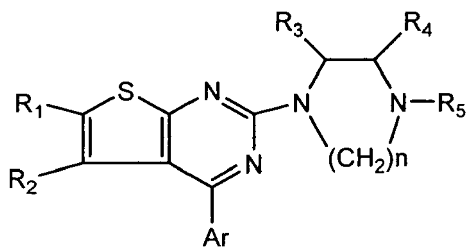

I

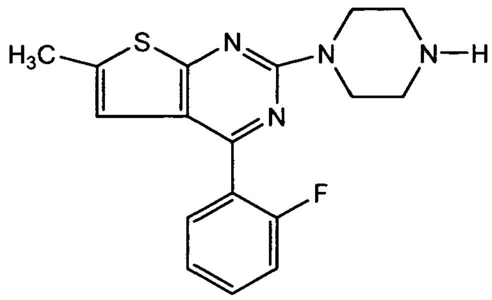

II

wherein, $R_1$ and $R_2$ independently represent hydrogen, halogen or a $C_1$-$C_6$ alkyl group; or $R_1$ and $R_2$ together with the carbon atom to which they are attached form a cycloalkylene group having 5 to 6 carbon atoms;
$R_3$ and $R_4$ independently represent hydrogen or a $C_1$-$C_6$ alkyl group;
$R_5$ is hydrogen, $C_1$-$C_6$ alkyl,

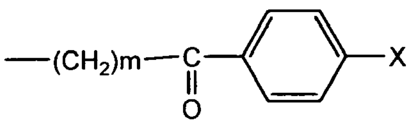

,

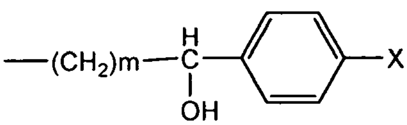

or -C(O)-NH-$R_6$
wherein m is an integer from about 1 to about 3, X is halogen and $R_6$ is a $C_1$-$C_6$ alkyl group; and
Ar is a substituted or unsubstituted phenyl, 2-thienyl or 3-thienyl group; and
n is 2 or 3; or a pharmaceutically acceptable salt thereof,
for the manufacture of a medicament for the treatment of

a functional bowel disorder, or
diarrhea predominant irritable bowel syndrome,

wherein the medicament is in unit dosage form and comprises from about 0.02 mg to about 200 mg of the compound or a pharmaceutically acceptable salt thereof.

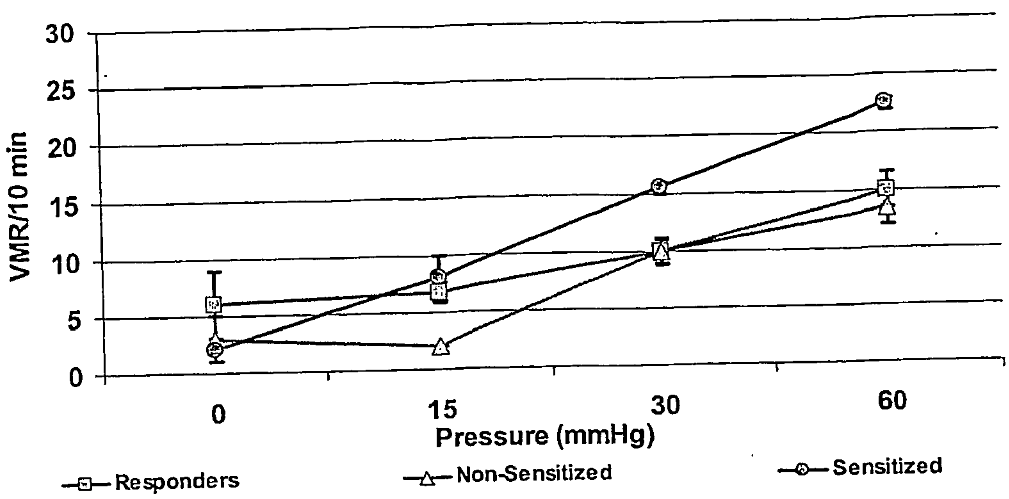
30
25
20
15
10
5
0
VMR/10 min
0
15
30
60
Pressure (mmHg)
Responders
Non-Sensitized
Sensitized

FIG. 1

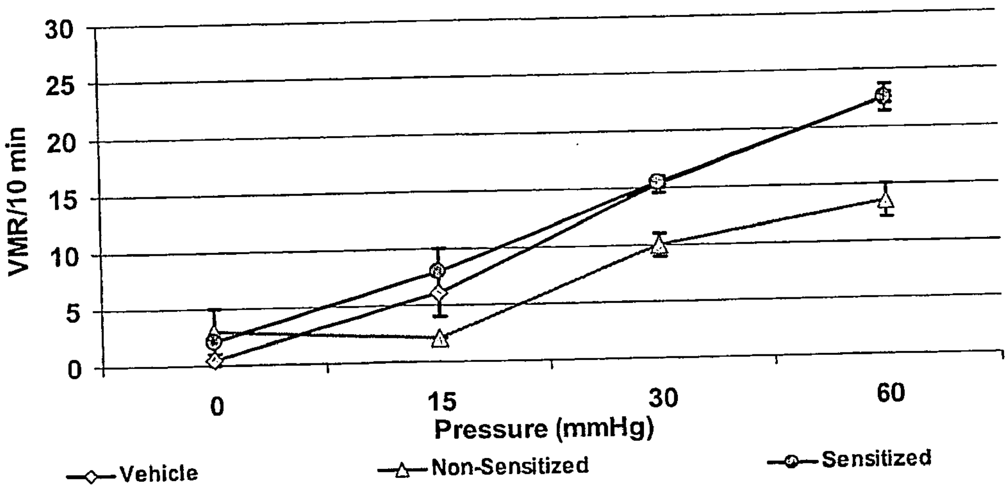
30
25
20
15
10
5
0
VMR/10 min
0
15
30
60
Pressure (mmHg)
Vehicle
Non-Sensitized
Sensitized

FIG. 2

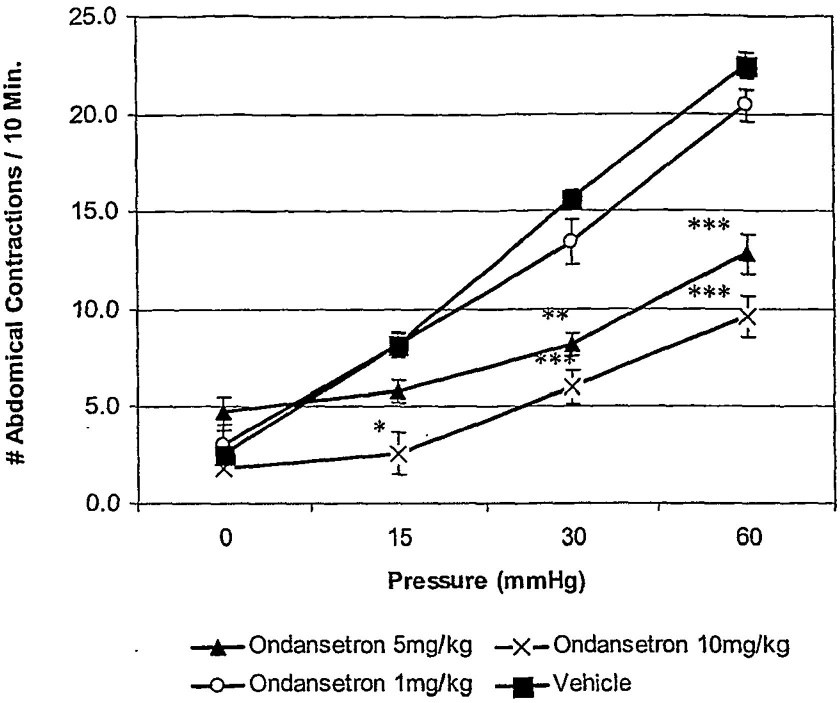
25.0
20.0
15.0
10.0
5.0
0.0
# Abdomical Contractions / 10 Min.
0
15
30
60
Pressure (mmHg)
*
**
***
***
***
Ondansetron 5mg/kg
Ondansetron 10mg/kg
Ondansetron 1mg/kg
Vehicle

FIG. 3

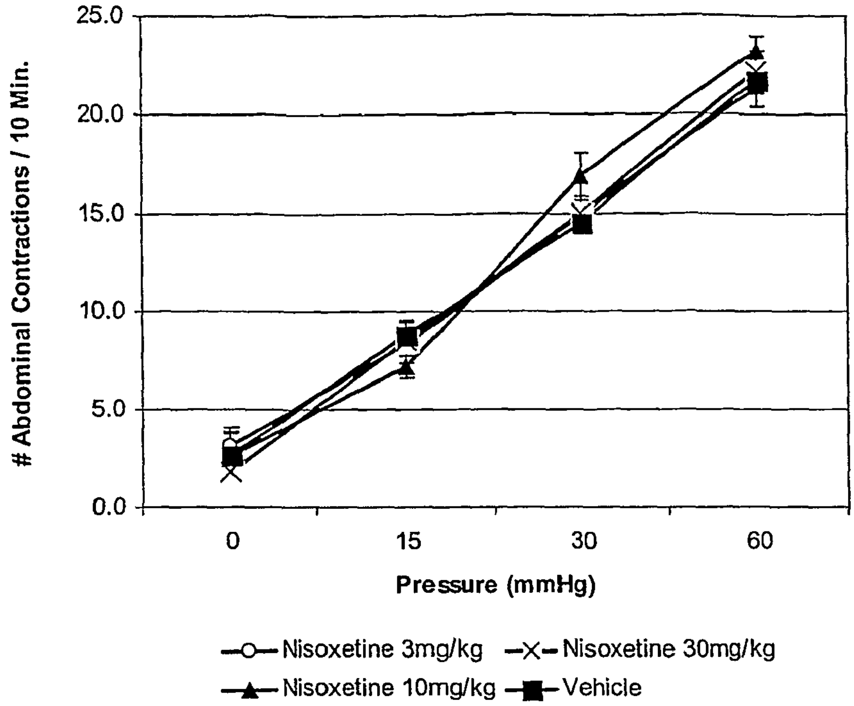
25.0
20.0
15.0
10.0
5.0
0.0
# Abdominal Contractions / 10 Min.
0
15
30
60
Pressure (mmHg)
Nisoxetine 3mg/kg
Nisoxetine 30mg/kg
Nisoxetine 10mg/kg
Vehicle

**FIG. 4**

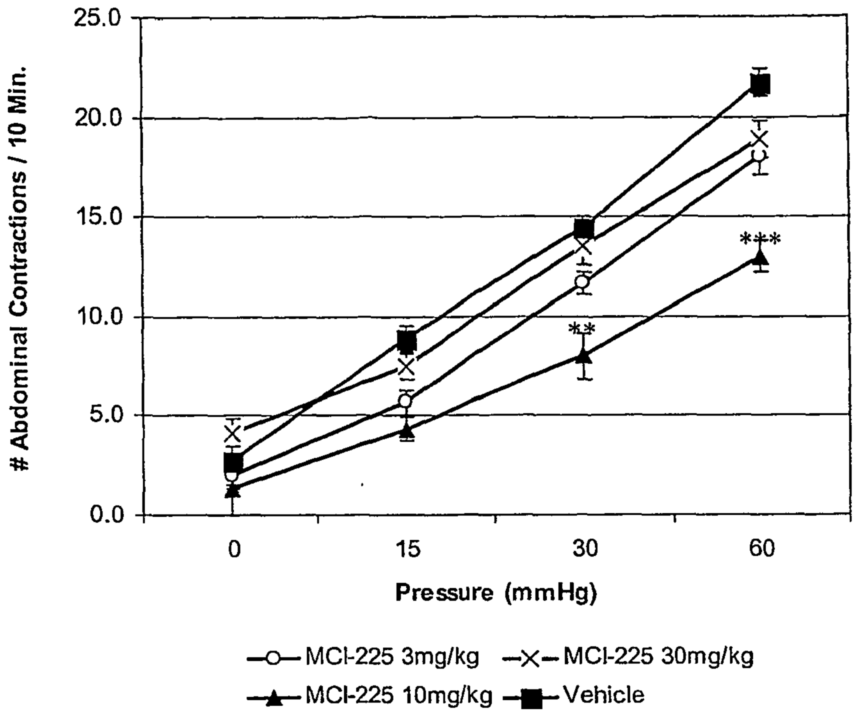
25.0
20.0
15.0
10.0
5.0
0.0
# Abdominal Contractions / 10 Min.
***
**
0
15
30
60
Pressure (mmHg)
MCI-225 3mg/kg
MCI-225 30mg/kg
MCI-225 10mg/kg
Vehicle

FIG. 5

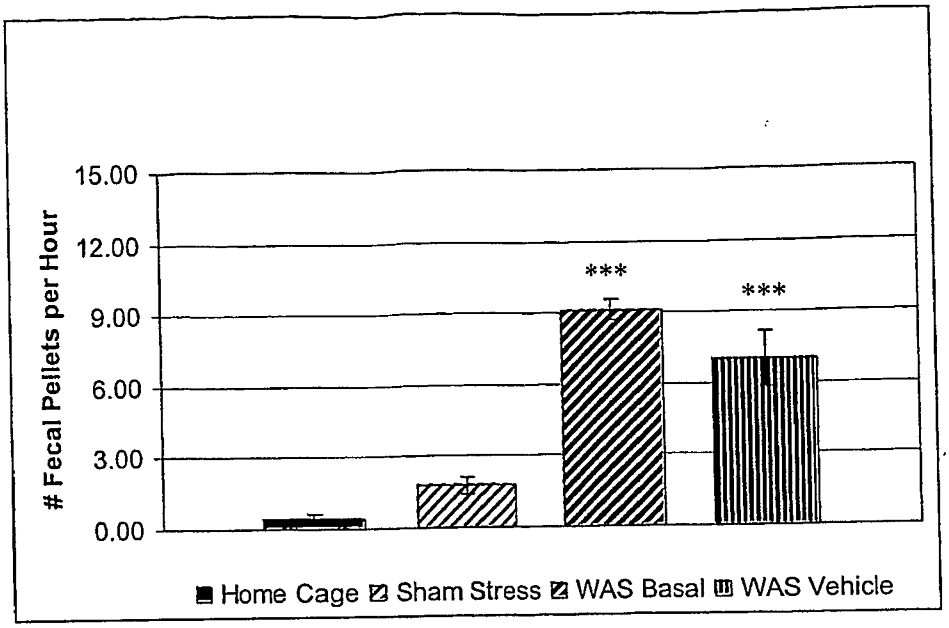

15.00
12.00
9.00
6.00
3.00
0.00
# Fecal Pellets per Hour
***
***
Home Cage
Sham Stress
WAS Basal
WAS Vehicle


FIG. 6

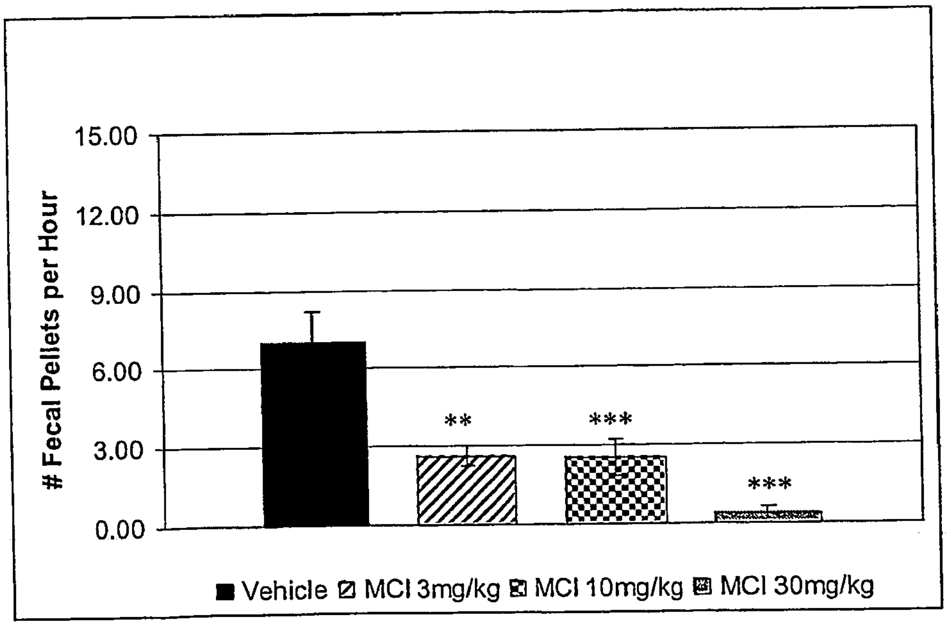

15.00
12.00
9.00
6.00
3.00
0.00
# Fecal Pellets per Hour
**
***
***
Vehicle
MCI 3mg/kg
MCI 10mg/kg
MCI 30mg/kg


FIG. 7

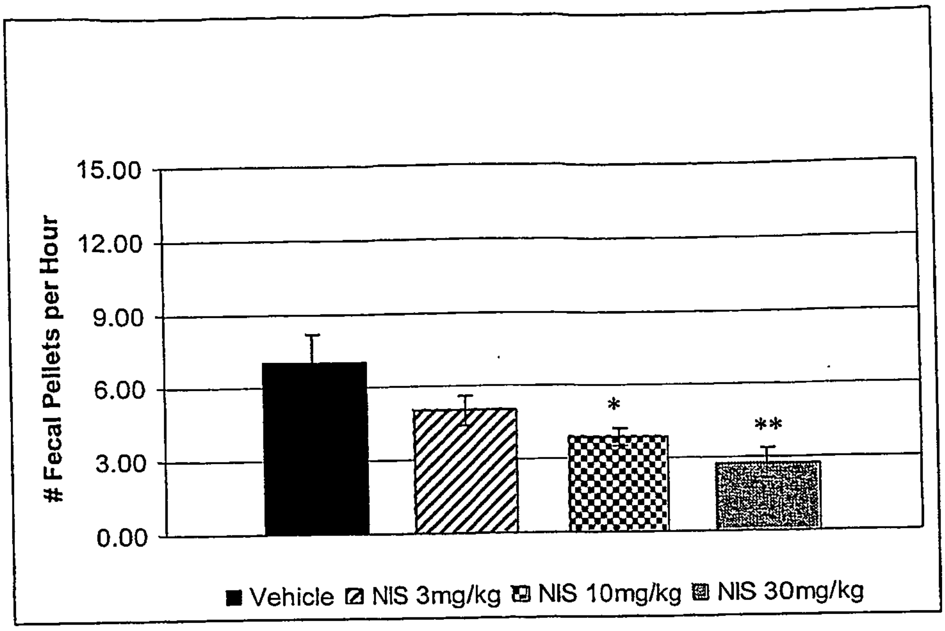
15.00
12.00
9.00
6.00
3.00
0.00
# Fecal Pellets per Hour
*
**
Vehicle NIS 3mg/kg NIS 10mg/kg NIS 30mg/kg

FIG. 8

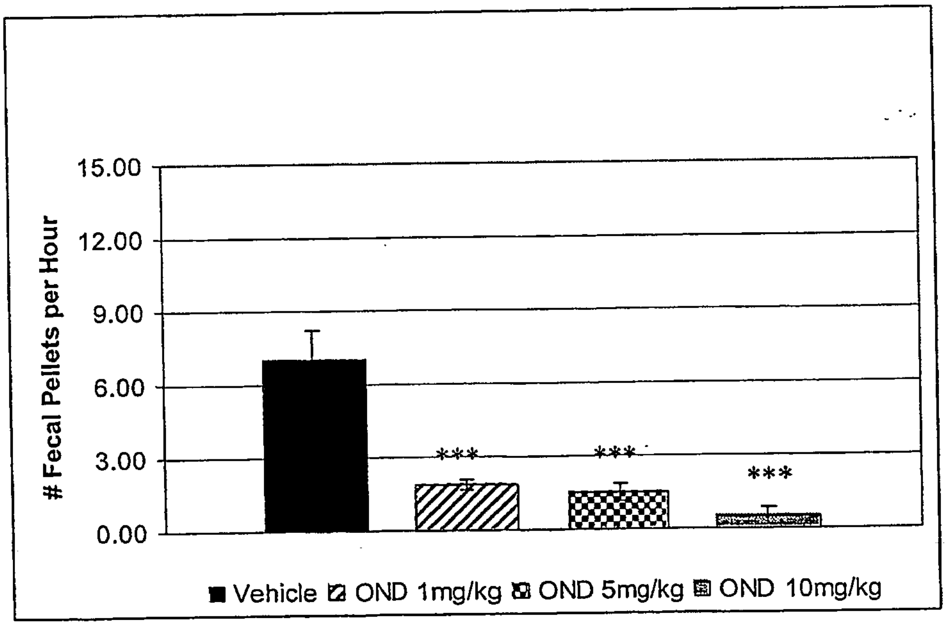
15.00
12.00
9.00
6.00
3.00
0.00
# Fecal Pellets per Hour
***
***
***
Vehicle OND 1mg/kg OND 5mg/kg OND 10mg/kg

FIG. 9

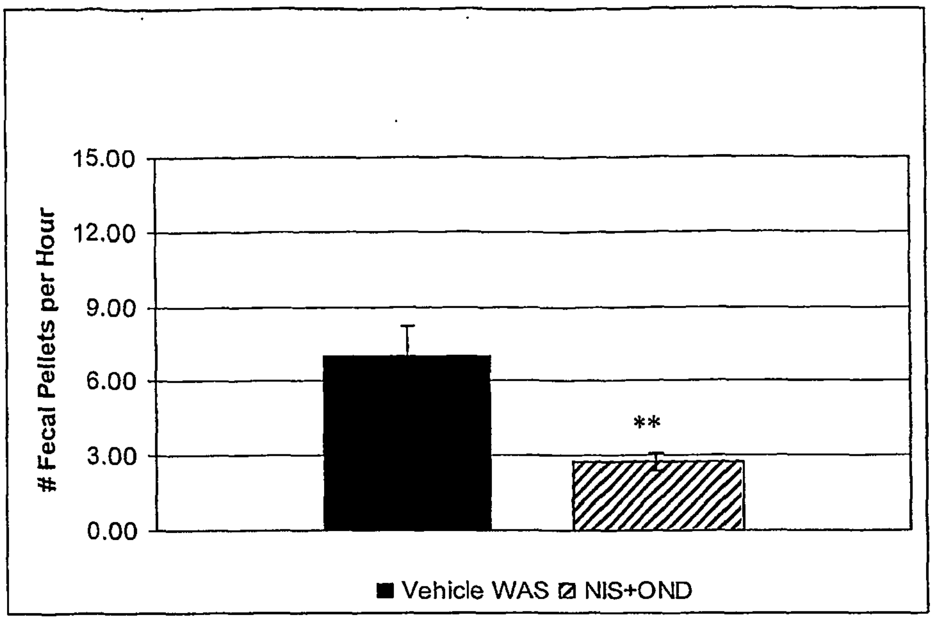
15.00
12.00
9.00
6.00
3.00
0.00
# Fecal Pellets per Hour
**
Vehicle WAS
NIS+OND

**FIG. 10**

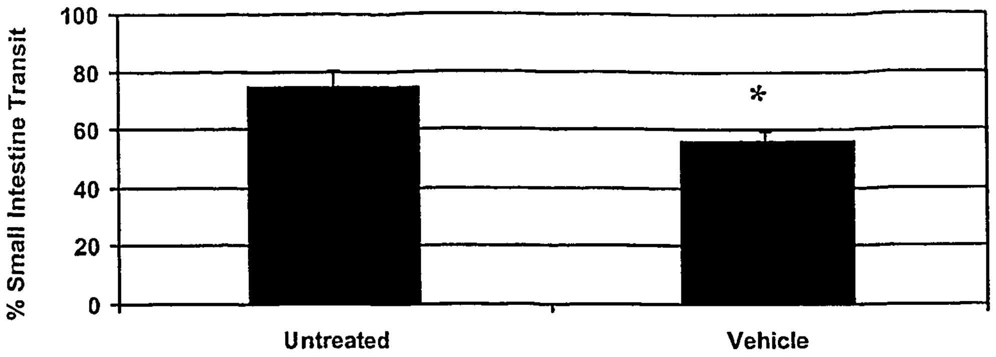
% Small Intestine Transit
100
80
60
40
20
0
*
Untreated
Vehicle

FIG. 11

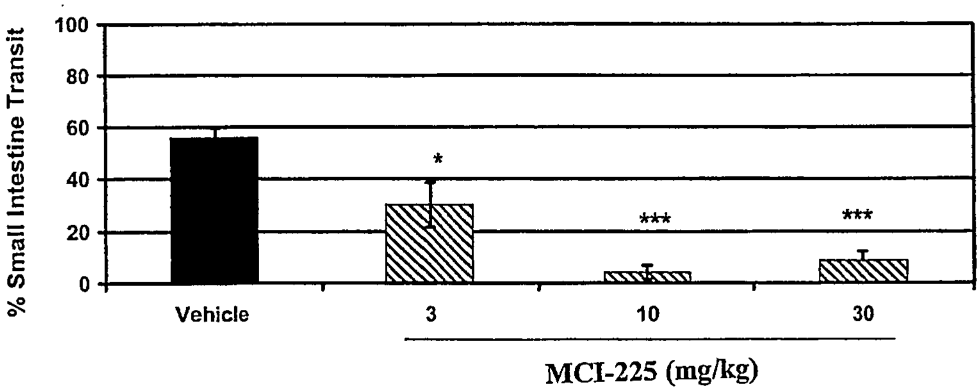
% Small Intestine Transit
100
80
60
40
20
0
*
***
***
Vehicle
3
10
30
MCI-225 (mg/kg)

FIG. 12

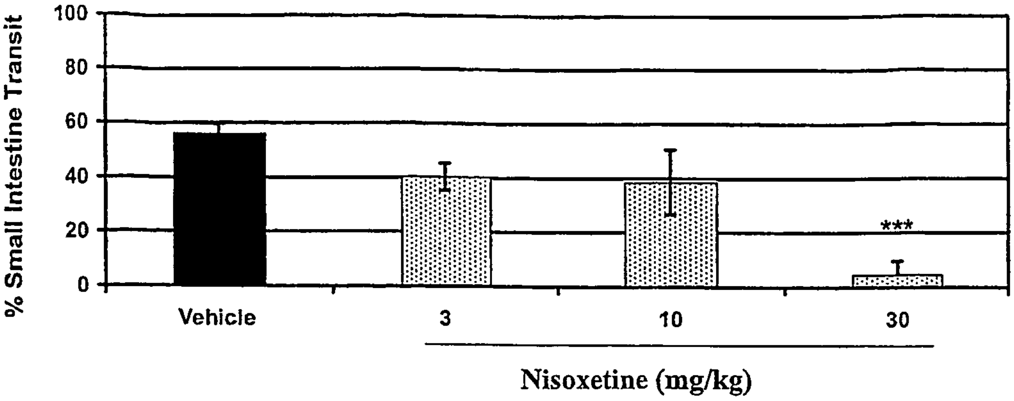
% Small Intestine Transit
100
80
60
40
20
0
***
Vehicle
3
10
30
Nisoxetine (mg/kg)

FIG. 13

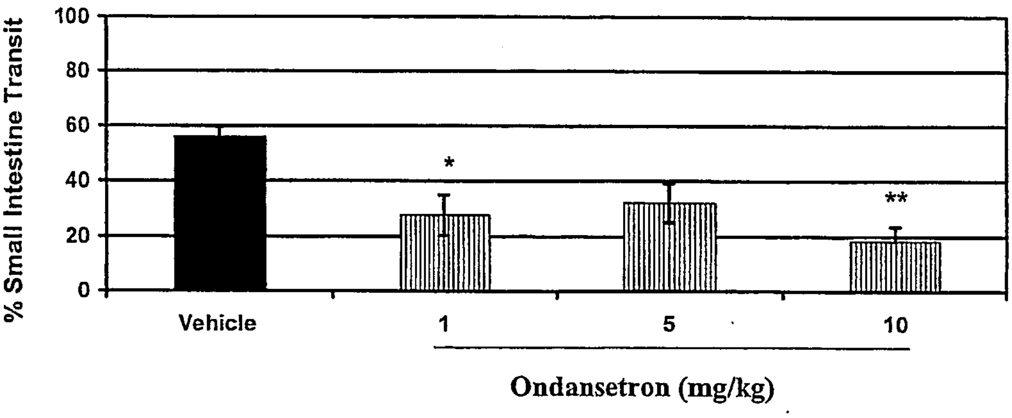
% Small Intestine Transit
100
80
60
40
20
0
*
**
Vehicle
1
5
10
Ondansetron (mg/kg)

FIG. 14

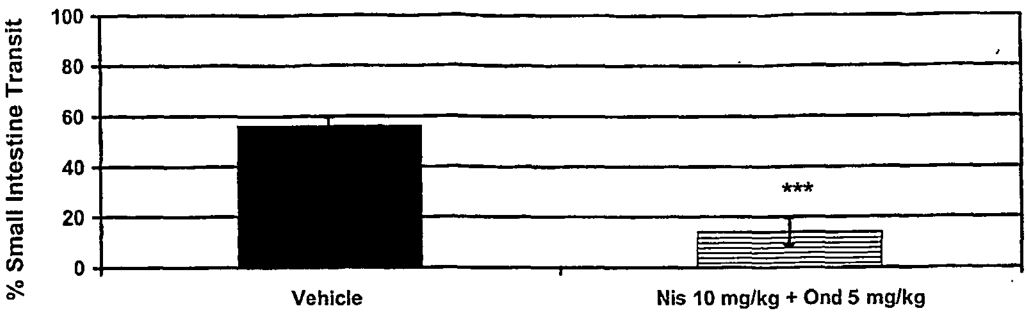
% Small Intestine Transit
100
80
60
40
20
0
***
Vehicle
Nis 10 mg/kg + Ond 5 mg/kg

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 49248003 P
- US 44007703 P
- US 4695568 A
- US 6340475 B
- US 6488962 B
- US 6451808 B
- US 5972389 A
- US 5582837 A
- US 5007790 A
- US 20030147952 A
- US 20030104062 A
- US 20030104053 A
- US 20030044466 A
- US 20030039688 A
- US 20020051820 A
- WO 0335041 A
- WO 0335040 A
- WO 0335029 A
- WO 0335177 A
- WO 0335039 A
- WO 0296404 A
- WO 0232416 A
- WO 0197783 A
- WO 0156544 A
- WO 0132217 A
- WO 9855107 A
- WO 9811879 A
- WO 9747285 A
- WO 9318755 A
- WO 9011757 A
- US 6448962 B
- US 20010018707 A
- US 20030029688 A
- WO 9626718 A
- WO 03035040 A


### Non-patent literature cited in the description


- **THOMPSON et al.** *Gut,* 1999, vol. 45 (II), II43-II47
- **CAMILLERI ; CHOI.** *Aliment. Pharm. Ther.,* 1997, vol. 11, 3-15
- **MAYER E.A. ; GEBHART, G.F.** Basic and Clinical Aspects of Chronic Abdominal Pain. Elsevier, 1993, vol. 9, 3-28
- **CLOUSE, R.R.** *Dig. Dis. Sci.,* 1994, vol. 39, 2352-2363
- **WOOD et al.** *Gut,* 1999, vol. 45 (II), II6-II16
- **EGUCHI et al.** *Arzneim.-Forschung/Drug Res.,* 1997, vol. 47 (12), 1337-47
- *Neuropharm.,* 1994, vol. 33, 261-273
- *Pharm. Rev.,* 1994, vol. 46, 157-203
- **G. KILPATRICK et al.** *Nature,* 1987, vol. 330, 746-748
- **BUTLER et al.** *Br. J. Pharmacol.,* 1988, vol. 94, 397-412
- **ITO et al.** *J. Pharmacol. Exp. Ther.,* 1997, vol. 280 (1), 67-72
- **GUNTER et al.** *Physiol. Behav.,* 2000, vol. 69 (3), 379-82
- **DEPOORTERE et al.** *J. Pharmacol. and Exp. Ther.,* 2000, vol. 294 (3), 983-990
- **MORTEAU et al.** *Fund. Clin. Pharmacol.,* 1994, vol. 8 (6), 553-62
- **GIBSON et al.** *Gastroenterology,* 2001, vol. 120 (5), A19-A20
- **GSCHOSSMANN et al.** *Eur. J. Gastro. Hepat.,* 2002, vol. 14 (10), 1067-72
- **YAUN et al.** *Br. J. Pharmacol.,* 1994, vol. 112 (4), 1095-1100
- **JIN et al.** *J. Pharm. Exp. Ther.,* 1999, vol. 288 (1), 93-97
- **VENKOVA et al.** *J. Pharm. Exp. Ther.,* 2002, vol. 300 (3), 1046-1052
- **MCRORIE et al.** *Dig. Dis. Sci.,* 1998, vol. 43, 957-963
- **KUGE et al.** *Dig. Dis. Sci.,* 2002, vol. 47, 2651-6
- **LANGLOIS et al.** *Eur. J. Pharmacol.,* 1996, vol. 318, 141-144
- **PLOURDE et al.** *Am. J. Physiol.,* 1997, vol. 273, G191-G196